# EUROPEAN PATENT SPECIFICATION

(11) **EP 3 606 529 B1**
(45) Date of publication and mention of the grant of the patent: **15.07.2026**
(21) Application number: 18718396.7
(22) Date of filing: 29.03.2018
(51) Int. Cl.: A61K 31/4747, A61K 31/13, A61K 45/06, A61P 25/14

(54) **(+)-ALPHA-DIHYDROTETRABENAZINE FOR USE IN THE TREATMENT A MOVEMENT DISORDER**
(+)-ALPHA-DIHYDROTETRABENAZINE ZUR BEHANDLUNG VON BEWEGUNGSSTÖRUNGEN
(+)-ALPHA-DIHYDROTETRABENAZINE POUR SON UTILISATION DANS LE TRAITEMENT DES TROUBLES DU MOUVEMENT

(30) Priority: 01.04.2017 GB 201705301; 28.04.2017 GB 201706816; 06.06.2017 US 201762515928 P; 06.06.2017 US 201762515937 P
(43) Date of publication of application: 12.02.2020
(73) Proprietor: Adeptio Pharmaceuticals Limited, London SW1Y 4LB (GB)
(72) Inventor: DUFFIELD, Andrew John, London SW1Y 4LB (GB); PANDYA, Anant, London SW1Y 4LB (GB)
(74) Representative: Schlich
(86) International application number: PCT/EP2018/058069
(87) International publication number: WO 2018/178233

(56) References cited:
- WO-A1-2015/171802
- WO-A2-2006/053067
- WO-A2-2015/120110
- WO-A2-2016/210180
- SARAH CATHERINE WALPOLE ET AL: "The weight of nations: an estimation of adult human biomass", BMC PUBLIC HEALTH, BIOMED CENTRAL, LONDON, GB, vol. 12, no. 1, 18 June 2012 (2012-06-18), pages 439, XP021132763, ISSN: 1471-2458, DOI: 10.1186/1471-2458-12-439
- ANONYMOUS: "Neurocrine Announces Phase II Results of VMAT2 Inhibitor NBI-98854 for Treatment of Tardive Dyskinesia", NEUROCRINE BIOSCIENCES, INC., 26 March 2012 (2012-03-26), San Diego, USA, XP093315456, Retrieved from the Internet <URL:https://www.prnewswire.com/news-releases/neurocrine-announces-phase-ii-results-of-vmat2-inhibitor-nbi-98854-for-treatment-of-tardive-dyskinesia-144264365.html>
- ANONYMOUS: "Neurocrine Announces Phase IIb Results Of VMAT2 Inhibitor NBI-98854 For Treatment Of Tardive Dyskinesia", NEUROCRINE BIOSCIENCES, INC., 9 September 2013 (2013-09-09), San Diego, USA, XP093315514, Retrieved from the Internet <URL:https://www.prnewswire.com/news-releases/neurocrine-announces-phase-iib-results-of-vmat2-inhibitor-nbi-98854-for-treatment-of-tardive-dyskinesia-223015671.html>
- ANONYMOUS: "Archive History for NCT02844179 (+)-Alpha-Dihydrotetrabenazine Phase I", 25 July 2016 (2016-07-25), XP055483994, Retrieved from the Internet <URL:https://clinicaltrials.gov/ct2/history/NCT02844179?V_1=View#StudyPageTop> [retrieved on 20180613]

## Description

This invention relates to the use of low doses of (+)-α-dihydrotetrabenazine for the treatment of movement disorders, such as Tourette's syndrome.

### Background of the Invention

Movement disorders can generally be classified into two categories: hyperkinetic movement disorders and hypokinetic movement disorders. Hyperkinetic movement disorders are caused by an increase in muscular activity and can cause abnormal and/or excessive movements, including tremors, dystonia, chorea, tics, myoclonus and stereotypies.

Hyperkinetic movement disorders are often psychological in nature and arise through improper regulation of amine neurotransmitters in the basal ganglia.

A particular hyperkinetic movement disorder is Tourette's syndrome, which is an inherited neurological condition characterised by multiple physical and vocal tics. The tics are usually repetitive, but random, physical movements or vocal noises. The vocal tics can be of various forms and include repeating one's own words, the words of others or other sounds. Onset usually occurs in children and continues through to adolescence and adulthood.

While the tics associated with Tourette's syndrome are temporarily suppressible, those affected can usually only suppress their tics for limited time periods. There is yet to be an effective treatment to cover all types of tics in all patients, but certain medicaments for tic suppression have been developed.

It is known that dopamine receptor antagonists display an ability to suppress tics in Tourette's syndrome patients and a number of dopamine receptor antagonists are currently used in the suppression of Tourette's tics, such as fluphenazine, risperidone, haloperidol and pimozide.

Type 2 vesicular monoamine transporter (VMAT2) is a membrane protein responsible for the transportation of monoamine neurotransmitters, such as dopamine, serotonin and histamine, from cellular cytosol into synaptic vesicles. Inhibition of this protein hinders presynaptic neurons from releasing dopamine, resulting in a depletion of dopamine levels in the brain.

VMAT2 inhibitors can be used to treat the symptoms of Tourette's syndrome.

Tetrabenazine (Chemical name: 1,3,4,6,7,11b-hexahydro-9,10-dimethoxy-3-(2-methylpropyl)-2H-benzo(a)quinolizin-2-one) has been in use as a pharmaceutical drug since the late 1950s. Initially used as an anti-psychotic, tetrabenazine is currently used for treating hyperkinetic movement disorders such as Huntington's disease, hemiballismus, senile chorea, tic, tardive dyskinesia and Tourette's syndrome, see for example Jankovic et al., Am. J. Psychiatry. (1999) Aug; 156(8):1279-81 and Jankovic et al., Neurology (1997) Feb; 48(2):358-62.

The primary pharmacological action of tetrabenazine is to reduce the supply of monoamines (e.g. dopamine, serotonin, and norepinephrine) in the central nervous system by inhibiting the human vesicular monoamine transporter isoform 2 (hVMAT2). The drug also blocks post-synaptic dopamine receptors.

The central effects of tetrabenazine closely resemble those of reserpine, but it differs from reserpine in that it lacks activity at the VMAT1 transporter. The lack of activity at the VMAT1 transporter means that tetrabenazine has less peripheral activity than reserpine and consequently does not produce VMAT1-related side effects such as hypotension.

Tetrabenazine is an effective and safe drug for the treatment of a variety of hyperkinetic movement disorders and, in contrast to typical neuroleptics, has not been demonstrated to cause tardive dyskinesia. Nevertheless, tetrabenazine does exhibit a number of dose-related side effects including causing depression, parkinsonism, drowsiness, nervousness or anxiety, insomnia and, in rare cases, neuroleptic malignant syndrome, see for example the introductory section of WO2016/127133 (Neurocrine Biosciences).

The chemical structure of tetrabenazine is as shown below. 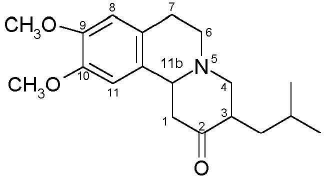 Structure of tetrabenazine

The compound has chiral centres at the 3 and 11b carbon atoms and hence can, theoretically, exist in a total of four isomeric forms, as shown below. 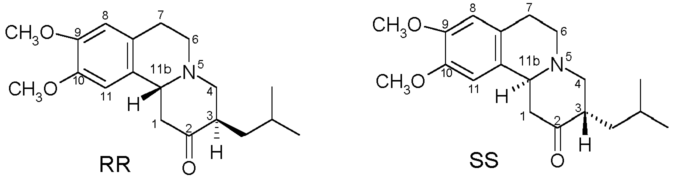 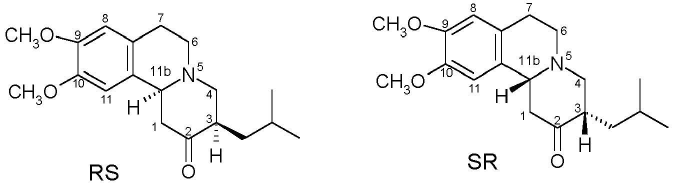 Possible tetrabenazine isomers

The stereochemistry of each isomer is defined using the "R and S" nomenclature developed by Cahn, Ingold and Prelog, see Advanced Organic Chemistry by Jerry March, 4th Edition, John Wiley & Sons, New York, 1992, pages 109-114. In this patent application, the designations "R" or "S" are given in the order of the position numbers of the carbon atoms. Thus, for example, *RS* is a shorthand notation for 3*R*,11b*S.* Similarly, when three chiral centres are present, as in the dihydrotetrabenazines described below, the designations *"R"* or *"S"* are listed in the order of the carbon atoms 2, 3 and 11b. Thus, the *2R,3S, 11bS* isomer is referred to in short hand form as *RSS* and so on.

Commercially available tetrabenazine is a racemic mixture of the *RR* and *SS* isomers and it would appear that the *RR* and *SS* isomers are the most thermodynamically stable isomers.

Tetrabenazine has somewhat poor and variable bioavailability. It is extensively metabolised by first-pass metabolism, and little or no unchanged tetrabenazine is typically detected in the urine. It is known that at least some of the metabolites of tetrabenazine are dihydrotetrabenazines formed by reduction of the 2-keto group in tetrabenazine.

Dihydrotetrabenazine (Chemical name: 2-hydroxy-3-(2-methylpropyl)-1,3,4,6,7,11b-hexahydro-9,10-dimethoxy-benzo(a)quinolizine) has three chiral centres and can therefore exist in any of the following eight optical isomeric forms: 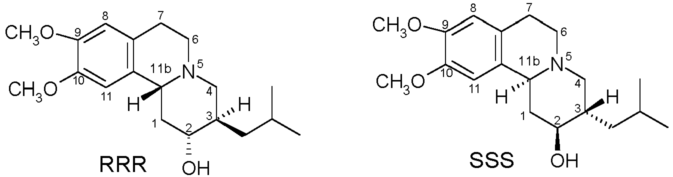 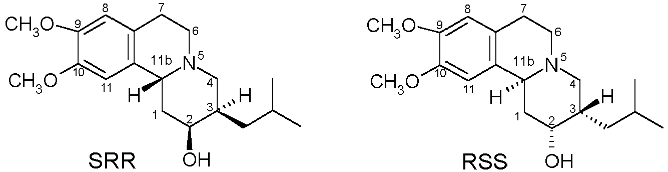 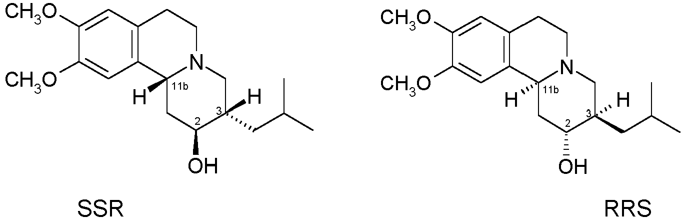 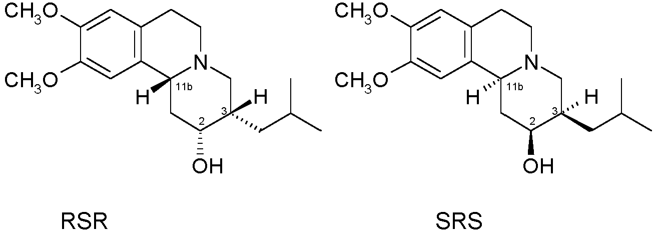 Dihydrotetrabenazine isomers

The synthesis and characterisation of all eight dihydrotetrabenazine isomers is described by Sun et al. (Eur. J. Med. Chem. (2011), 1841-1848).

Of the eight dihydrotetrabenazine isomers, four isomers are derived from the more stable RR and SS isomers of the parent tetrabenazine, namely the RRR, SSS, SRR and RSS isomers.

The RRR and SSS isomers are commonly referred to as "alpha (α)" dihydrotetrabenazines and can be referred to individually as (+)-α-dihydrotetrabenazine and (-)-α-dihydrotetrabenazine respectively. The alpha isomers are characterised by a *trans* relative orientation of the hydroxyl and 2-methylpropyl substituents at the 2- and 3-positions - see for example, Kilbourn et al., Chirality, 9:59-62 (1997) and Brossi et al., Helv. Chim. Acta., vol. XLI, No. 193, pp1793-1806 (1958).

The SRR and RSS isomers are commonly referred to as "beta (β)" isomers and can be referred to individually as (+)-β-dihydrotetrabenazine and (-)-β-dihydrotetrabenazine respectively. The beta isomers are characterised by a *cis* relative orientation of the hydroxyl and 2-methylpropyl substituents at the 2- and 3-positions.

Although dihydrotetrabenazine is believed to be primarily responsible for the activity of the drug, there have been no studies published to date that contain evidence demonstrating which of the various stereoisomers of dihydrotetrabenazine is responsible for its biological activity. More specifically, there have been no published studies demonstrating which of the stereoisomers is responsible for the ability of tetrabenazine to treat movement disorders such as Tourette's syndrome.

Schwartz et al. (Biochem. Pharmacol. (1966), 15: 645-655) describes metabolic studies of tetrabenazine carried out in rabbits, dogs and humans. Schwartz *et al.* identified nine metabolites, five of which were unconjugated and the other four of which were conjugated with glucuronic acid. The five unconjugated metabolites were the alpha- and beta-dihydrotetrabenazines, their two oxidised analogues in which a hydroxyl group has been introduced into the 2-methylpropyl side chain, and oxidised tetrabenazine in which a hydroxyl group has been introduced into the 2-methylpropyl side chain. The four conjugated metabolites were all compounds in which the 9-methoxy group had been demethylated to give a 9-hydroxy compound. The chirality of the various metabolites was not studied and, in particular, there was no disclosure of the chirality of the individual α- and β-isomers.

Scherman et al., (Mol. Pharmacol. (1987), 33, 72-77 describes the stereospecificity of VMAT2 binding between racemic α- and β- dihydrotetrabenazine. They reported that α-dihydrotetrabenazine had a 3- to 4-fold higher affinity for the Chromaffin Granule Monoamine Transporter than the β-isomer, when studied in vitro. However, Scherman *et al.* does not disclose the resolution or testing of the individual enantiomers of the α- and β-dihydrotetrabenazines.

Mehvar et al. (J. Pharm. Sci. (1987), 76(6), 461-465) reported a study of the concentrations of tetrabenazine and dihydrotetrabenazine in the brains of rats following administration of either tetrabenazine or dihydrotetrabenazine. The study showed that despite its greater polarity, dihydrotetrabenazine was able to cross the blood-brain barrier. However, the stereochemistry of the dihydrotetrabenazine was not disclosed.

Mehvar et al. (Drug Metabolism and Disposition (1987), 15:2, 250-255) describes studies of the pharmacokinetics of tetrabenazine and dihydrotetrabenazine following administration of tetrabenazine to four patients affected by tardive dyskinesia. Oral administration of tetrabenazine resulted in low plasma concentrations of tetrabenazine but relatively high concentrations of dihydrotetrabenazine. However, the stereochemistry of the dihydrotetrabenazine formed *in vivo* was not reported.

Roberts et al. (Eur. J. Clin. Pharmacol. (1986), 29: 703-708) describes the pharmacokinetics of tetrabenazine and its hydroxy-metabolite in patients treated for involuntary movement disorders. Roberts *et al.* reported that tetrabenazine was extensively metabolised after oral administration resulting in very low plasma concentrations of tetrabenazine but much higher concentrations of a hydroxy-metabolite. Although they did not describe the identity of the hydroxy-metabolites, they suggested that the high plasma concentrations of the hydroxy-metabolites may be therapeutically important (since the metabolites were known to be pharmacologically active) and that, in view of the disclosure in Schwartz *et al.* (*idem*), the combination of *cis* and *trans* isomers (i.e. beta and alpha isomers) could be more therapeutically important than the parent drug.

Michael Kilbourn and collaborators at the University of Michigan Medical School have published a number of studies relating to the various isomers of dihydrotetrabenazines. Thus, in Med. Chem. Res. (1994), 5:113-126, Kilbourn *et al*. describe the use (+/-)-α-[11C]-dihydrotetrabenazine as *in vivo* imaging agents for VMAT2 binding studies.

In Eur. J. Pharmacol (1995) 278, 249-252, Kilbourn *et al.* reported competition binding studies using [³H]-tetrabenazine to study the *in vitro* binding affinity of (+)-, (-)-, and (+/-)-α-DHTBZ. The binding assays gave Ki values of 0.97 nM for (+)-α-dihydrotetrabenazine and 2.2 µM for (-)-α-dihydrotetrabenazine, thereby showing that the (+) alpha isomer has much greater binding affinity for the VMAT2 receptor than the (-) alpha isomer. However, no studies were reported, or conclusions drawn, as to the usefulness of either isomer in the treatment of movement disorders such as Tourette's syndrome.

In Chirality (1997) 9:59-62, Kilbourn *et al.* described studies aimed at identifying the absolute configuration of (+)-α-dihydrotetrabenazine from which they concluded that it has the 2R, 3R, 11b*R* configuration shown above. They also referred to the Schwartz *et al.* and Mehvar *et al.* articles discussed above as indicating that the α- and β-dihydrotetrabenazines are likely to be the pharmacologically active agents in the human brain but they drew no explicit conclusions as to the precise stereochemical identities of the active metabolites of tetrabenazine.

In Synapse (2002), 43:188-194, Kilbourn *et al.* described the use of (+)-α-[¹¹C]-dihydrotetrabenazine as an agent used to measure specific *in vivo* binding of the VMAT receptor, in "infusion to equilibrium methods". They found that (-)-α-[¹¹C]-dihydrotetrabenazine produced a uniform brain distribution, consistent with the earlier observations that this enantiomer has a low VMAT affinity.

Sun *et al.* (*idem*) investigated the VMAT2 binding affinities of all eight dihydrotetrabenazine isomers. They found that all of the dextrorotatory enantiomers exhibited dramatically more potent VMAT2 binding activity than their corresponding laevorotatory enantiomers with the most active (+)-α-isomer being found to be the most active. However, Sun *et al.* did not carry out any investigations into the relative efficacies of the individual isomers in treating movement disorders such as Tourette's syndrome.

WO2015/120110 (Auspex) describes extended-release formulations that can contain any of a wide variety of different pharmacological agents, including tetrabenazine and dihydrotetrabenazine. WO2015/120110 discloses in general terms that the formulations may comprise between 5mg and 30mg of tetrabenazine or dihydrotetrabenazine and more specifically discloses extended-release formulations comprising tetrabenazine or dihydrotetrabenazine in amounts of 7.5 mg, 12.5 mg, 15 mg, 25 mg, 30 mg and 50 mg. However, there is no specific disclosure of formulations containing such amounts of (+)-α-dihydrotetrabenazine.

Furthermore, there are no worked examples of any dihydrotetrabenazine formulations; but only formulations containing tetrabenazine.

WO 2006/053067 (Prestwick) describes the use of combinations of amantadine and a tetrabenazine compound (which can be tetrabenazine or dihydrotetrabenazine) for treating hyperkinetic movement disorders. WO 2006/053067 discloses that the amount of dihydrotetrabenazine administered may be 10-400 mg per day (although no examples are provided showing that the doses at the lower ends of these ranges are effective) and pharmaceutical compositions containing as little as 10 mg are also described. WO 2006/053067 does not specifically link any particular isomers of dihydrotetrabenazine to these amounts and, more particularly, does not disclose the specific use of 10mg of (+)-α-dihydrotetrabenazine. No experimental data are provided in WO 2006/053067.

WO 2011/153157 (Auspex Pharmaceutical, Inc.) describes deuterated forms of dihydrotetrabenazine. Many deuterated forms of dihydrotetrabenazine are depicted but the application only provides sufficient information to allow a small number of the depicted compounds to be synthesised. Although racemic mixtures of d₆-α-dihydrotetrabenazine and d₆-β-dihydrotetrabenazine are disclosed, these mixtures were not resolved and the properties of the individual (+) and (-) isomers were not studied. Similarly, WO 2014/047167 (Auspex Pharmaceutical, Inc.) describes a number of deuterated forms of tetrabenazine and its derivatives. Again, the individual (+) and (-) isomers of deuterated forms of α- and β-dihydrotetrabenazine were not separated or studied.

It appears therefore that, up to the present, it remains unclear as to precisely which dihydrotetrabenazine isomers are responsible for the therapeutic properties resulting from the administration of tetrabenazine.

It has also remained somewhat unclear up until now whether (+)-α-dihydrotetrabenazine will provide a therapeutically useful effect in the treatment of movement disorders such as Tourette's syndrome without the accompaniment of unwanted side effects such as those described above. Thus, for example, whereas WO2016/127133 (Neurocrine Biosciences) refers to the Kilbourn *et al.* article in Chirality (*idem*) as indicating that (+)-α- dihydrotetrabenazine is the active metabolite of tetrabenazine. WO2016/127133, it also refers to the studies reported in Login et al. (1982), Ann. Neurology 12:257-62 and Reches et al., J. Pharmacol. Exp. Ther. (1983), 225:515-521 which indicate that tetrabenazine inhibits presynaptic and postsynaptic dopamine receptors in the rat brain. It is suggested in WO2016/127133 that this "off-target" activity of tetrabenazine may be responsible for some of the observed side effects of tetrabenazine.

As discussed above, it is known that tetrabenazine exhibits a number of dose-related side effects including causing depression and parkinsonism (see WO2016/127133). It appears that these side-effects may also be caused by VMAT2 inhibition and that consequently it is difficult to separate the therapeutic effect of tetrabenazine and tetrabenazine-derived compounds from these side-effects (see Müller, "Valbenazine granted breakthrough drug status for treating tardive dyskinesia", Expert Opin. Investig. Drugs (2015), 24(6), pp. 737 - 742).

In an attempt to avoid or reduce the side-effects associated with tetrabenazine, a valine ester prodrug of (+)-α-dihydrotetrabenazine has been developed, known by its INN name, Valbenazine. The structure of Valbenazine is shown below:

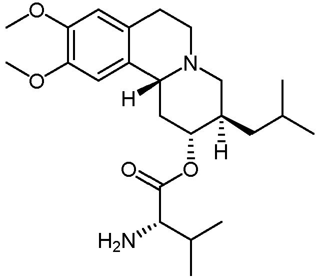

As disclosed in US8039627, Valbenazine is prepared by reacting (+)-α-dihydrotetrabenazine with carbobenzyloxy-L-valine in dichloromethane and 4-dimethylaminopyridine (DMAP) in the presence of N,N'-dicyclohexylcarbodiimide (DCC) to give the intermediate 2-benzyloxycarbonylamino-3-methyl-butyric acid (2R,3R,1bR)-3-isobutyl-9,10-dimethoxy-1,3,4,6,7,11b-hexahydro-2H-pyrido[2,1-a]isoquinolin-2-yl ester. The intermediate is then hydrogenated over palladium on carbon to remove the benzyloxycarbonyl protecting group to give Valbenazine.

Müller ("Valbenazine granted breakthrough drug status for treating tardive dyskinesia", Expert Opin. Investig. Drugs (2015), 24(6), pp. 737 - 742) describes a Phase Ilb clinical study of Valbenazine ("KINECT 1") in patients suffering from tardive dyskinesia. Although some reduction of symptoms was observed when doses of Valbenazine at 100 mg/day (equivalent to about 76 mg of (+)-α-dihydrotetrabenazine) were observed, subjects who received 50 mg/day of Valbenazine (equivalent to about 38 mg of (+)-α-dihydrotetrabenazine) did not show any significant signs of improvement, when scored with the abnormal involuntary movement scale (AIMS). Müller concluded that this study was more or less a failure, probably due to low Valbenazine dosing.

In a further study ("KINECT 2") described in the same paper, subjects were initially dosed at 25 mg/day, with the dose range increasing to 75 mg/day. By the end of the study, when measurements were taken, 21 out of 34 of the subjects treated with Valbenazine were being dosed at 75 mg/day (O'Brien et al, "Kinect 2: NBI-98854 treatment of moderate to severe tardive dyskinesia" Mov. Disord. 2014;29 (Suppl 1):829). The analysis does not provide a breakdown of the reduction in abnormal involuntary movements in patients who were being treated with 75 mg/day by the end of the trial and those who were being treated with 25mg/day or 50mg/day by the end of the trial.

A further Phase III trial of Valbenazine, reported by O'Brien et al ("KINECT 3 A randomised, Double-Blind Placebo-Controlled Phase 3 Trial of Valbenazine (NBI-98854) for Tardive Dyskinesia (PL02.003)", Neurology (2016), 86(16), Supplement PL02.003) investigated the change in abnormal involuntary movements in Tardive Dyskinesia sufferers when administered with 40mg or 80mg of Valbenazine per day. It was found that 80mg/day of Valbenazine resulted in a significant improvement in the Abnormal Involuntary Movement Score and it was concluded that 80mg/day Valbenazine was associated with a significant improvement in Tardive Dyskinesia.

WO 2015/171802 (Neurocrine Biosciences, Inc.) describes methods for treating hyperkinetic diseases by administering therapeutic agents that produce plasma concentrations of (+)-α-dihydrotetrabenazine such that there is a Cₘₐₓ of between about 15ng/ml and 60 ng/ml and a Cₘᵢₙ of at least 15 ng/ml over an eight hour period. Although it is suggested in WO 2015/171802 that this can be accomplished by administering (+)-α-dihydrotetrabenazine *per se,* the experiments described in WO 2015/171802 only provide data for (+)-α-dihydrotetrabenazine levels achieved after the administration of Valbenazine. In Example 1 of WO 2015/171802, it is concluded that a concentration of 30 ng/ml of (+)-α-dihydrotetrabenazine in plasma is an appropriate target and that exposures below 15 ng/ml are suboptimal across the general tardive dyskinesia (TD) population. In Example 2 of WO 2015/171802, it is disclosed that a 50 mg dose of Valbenazine appeared to maintain the required plasma levels of (+)-α-dihydrotetrabenazine.

WO2016/210180 (Neurocrine Biosciences) discloses the use of VMAT2 inhibitors for treating various neurological disorders. (+)-α-dihydrotetrabenazine is mentioned as an example of a VMAT2 inhibitor and therapeutic agents that provide plasma concentrations similar to those disclosed in WO2015/171802 are disclosed. The VMAT2 inhibitory compounds specifically exemplified in WO2016/210180 are Valbenazine and [(2R, 3S, 11b*R*)-9,10-dimethoxy-3-(2-methylpropyl)-1H,2H,3H,4H,6H,7H,11bH-pyrido[2,1-a]isoquinolin-2-yl]methanol.

The website https://clinicaltrials.gov/study/NCT02844179 discloses a clinical trial towards initial safety and tolerability of single oral doses of (+)-α-dihydrotetrabenaxine in normal volunteers.

### The Invention

Investigations made by the present applicant indicate that (+)-α-dihydrotetrabenazine *per se* having the chemical name, "(*R,R,R*)-3-isobutyl-9,10-dimethyloxy-1,3,4,5,7,11b-hexahydro-2H-pyrido[2,1-a]isoquinolin-2-ol", and having the formula (I) 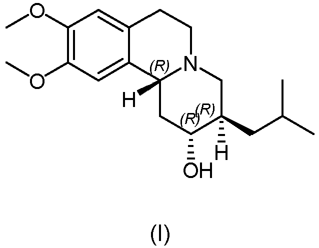 is effective in the treatment of movement disorders at much lower doses than could have been predicted from the literature (for example from WO 2015/171802) and that its use at such lower doses can avoid or minimize the unwanted side effects associated with tetrabenazine.

More particularly, experiments carried out by the present inventors indicate that movement disorders such as Tourette's syndrome can be treated effectively by administering much lower doses of (+)-α-dihydrotetrabenazine *per se* than the doses of Valbenazine required in WO 2015/171802.

In addition, contrary to the teachings of WO 2015/171802, it has been found that by administering (+)-α-dihydrotetrabenazine at a dose to provide blood plasma concentrations that do not exceed 15 ng/mL for a period of 8 hours, efficacious results are achieved.

The use of lower dosages is expected to reduce the biological side-effects associated with tetrabenazine and tetrabenazine derivatives, including parkinsonism and depression.

An advantage of using (+)-α-dihydrotetrabenazine *per se* rather than the prodrug Valbenazine is that it avoids the two additional synthetic steps and additional purification step required to prepare Valbenazine from (+)-α-dihydrotetrabenazine. In addition, the release of the active (+)-α-dihydrotetrabenazine into the blood plasma is not limited by the rate of degradation of the prodrug.

A further unexpected benefit of using low doses of (+)-α-dihydrotetrabenazine is that experiments conducted by the present inventors suggest that whereas abnormal movements of the type found in movement disorders will be reduced or suppressed by the drug, normal movements will not be. This is in contrast to risperidone, a well-used treatment for movement disorders, where the levels of both normal and abnormal movements can be reduced by administration of the drug.

On the basis of these findings, it is envisaged that low doses of (+)-α-dihydrotetrabenazine will be useful in the prophylaxis or treatment of *inter alia* the disease states and conditions for which tetrabenazine is currently used or proposed. Thus, by way of example, and without limitation, it is envisaged that low doses of (+)-α-dihydrotetrabenazine may be used for the treatment of hyperkinetic movement disorders such as Huntington's disease, hemiballismus, senile chorea, tic disorders, tardive dyskinesia, dystonia and, in particular, Tourette's syndrome.

Accordingly, in a first Embodiment of the invention (Embodiment 1.1), there is provided (+)-α-dihydrotetrabenazine or a pharmaceutically acceptable salt thereof for use in treating a hyperkinetic movement disorder wherein the treatment comprises administering to a subject an effective amount of (+)-α-dihydrotetrabenazine or a pharmaceutical salt thereof, wherein the said effective amount corresponds to an amount of (+)-α-dihydrotetrabenazine free base of from 0.05 mg/kg to 0.3 mg/kg per day.

In particular embodiments of the invention, there are provided:
1.2 (+)-α-Dihydrotetrabenazine for use according to Embodiment 1.1 wherein the said effective amount corresponds to an amount of (+)-α-dihydrotetrabenazine free base of from 0.1 mg/kg to 0.2 mg/kg per day.

The amount of (+)-α-dihydrotetrabenazine or a pharmaceutically acceptable salt thereof administered may be determined by the desired level of blocking of the VMAT2 proteins. Accordingly, the invention further provides:
1.3 (+)-α-Dihydrotetrabenazine for use according to any one of Embodiments 1.1 or 1.2 wherein the effective amount of (+)-α-dihydrotetrabenazine or a pharmaceutically acceptable salt thereof administered to the subject is sufficient to cause a level of blocking of between 50% and 85% of VMAT2 proteins in the subject.
1.4 (+)-α-Dihydrotetrabenazine for use according to any one of Embodiments 1.1 to 1.3 wherein the effective amount of (+)-α-dihydrotetrabenazine or a pharmaceutically acceptable salt thereof administered to the subject is sufficient to cause a level of blocking of between 55% and 85% of VMAT2 proteins in the brain of the subject.
1.5 (+)-α-Dihydrotetrabenazine for use according to Embodiment 1.3 or Embodiment 1.4 which treatment comprises administering to the subject an amount of (+)-α-dihydrotetrabenazine or pharmaceutically acceptable salt thereof sufficient to cause a level of blocking of between 70% and 85% of the VMAT2 proteins in the subject or, as the case may be, in the brain of the subject.

In further Embodiments, the invention provides:
1.5 (+)-α-Dihydrotetrabenazine for use according to any one of Embodiments 1.1 to 1.4 wherein the (+)-α-dihydrotetrabenazine or pharmaceutically acceptable salt thereof is administered to the subject once per day.
1.6 (+)-α-Dihydrotetrabenazine for use according to any one of Embodiments 1.1 to 1.4 wherein the (+)-α-dihydrotetrabenazine or pharmaceutically acceptable salt thereof is administered to the subject twice per day.
1.7 (+)-α-Dihydrotetrabenazine for use according to any one of Embodiments 1.1 to 1.6 wherein the (+)-α-dihydrotetrabenazine or pharmaceutically acceptable salt thereof is not administered in combination with a therapeutically effective amount of amantadine.
1.8. (+)-α-Dihydrotetrabenazine for use according to Embodiment 1.7 wherein the (+)-α-dihydrotetrabenazine or pharmaceutically acceptable salt thereof is not administered in combination with any amount of amantadine.
1.9 (+)-α-Dihydrotetrabenazine for use according to any one of Embodiments 1.1 to 1.8 wherein the (+)-α-dihydrotetrabenazine or pharmaceutically acceptable salt thereof is administered as a non-extended or delayed release dosage form (e.g. an immediate release unit dosage form).
1.10 (+)-α-Dihydrotetrabenazine for use according to any one of Embodiments 1.1 to 1.9 wherein the (+)-α-dihydrotetrabenazine or pharmaceutically acceptable salt is administered orally.
1.11 (+)-α-Dihydrotetrabenazine for use according to Embodiment 1.10 wherein the (+)-α-dihydrotetrabenazine or pharmaceutically acceptable salt is administered in the form of a tablet, capsule, solution, syrup or suspension.
1.12 (+)-α-Dihydrotetrabenazine for use according to Embodiment 1.10 wherein the (+)-α-dihydrotetrabenazine or pharmaceutically acceptable salt is administered in the form of a tablet.
1.13 (+)-α-Dihydrotetrabenazine for use according to Embodiment 1.10 wherein the (+)-α-dihydrotetrabenazine or pharmaceutically acceptable salt is administered in the form of a solution.
1.14 (+)-α-Dihydrotetrabenazine for use according to Embodiment 1.10 wherein the (+)-α-dihydrotetrabenazine or pharmaceutically acceptable salt is administered in the form of a syrup.
1.15 (+)-α-Dihydrotetrabenazine for use according to Embodiment 1.10 wherein the (+)-α-dihydrotetrabenazine or pharmaceutically acceptable salt is administered in the form of a suspension.
1.16 (+)-α-Dihydrotetrabenazine for use according to Embodiment 1.10 wherein the (+)-α-dihydrotetrabenazine or pharmaceutically acceptable salt is administered in the form of a capsule.

The (+)-α-dihydrotetrabenazine is for use in the treatment of a hyperkinetic movement disorder such as Huntington's disease, hemiballismus, senile chorea, tic disorders, tardive dyskinesia, dystonia, myoclonus and Tourette's syndrome.

More particularly, the (+)-α-dihydrotetrabenazine is for use in the treatment of a hyperkinetic movement disorder selected from tic disorders, tardive dyskinesia and Tourette's syndrome.

In one particular embodiment, the (+)-α-dihydrotetrabenazine is for use in the treatment of tardive dyskinesia.

In another particular embodiment, the (+)-α-dihydrotetrabenazine is for use in the treatment of Tourette's syndrome.

The term "treatment" as used herein in the context of treating a condition or disorder, pertains generally to treatment and therapy in which some desired therapeutic effect is achieved, for example, the inhibition of the progress of the condition, and includes a reduction in the rate of progress, a halt in the rate of progress, amelioration of the condition, diminishment or alleviation of at least one symptom associated or caused by the condition being treated and cure of the condition. When the hyperkinetic movement disorder being treated is Tourette's Syndrome, treatment of the disorder may pertain to a reduction of the incidence or severity of tics.

Accordingly, in further embodiments, the invention also provides:
1.17 (+)-α-Dihydrotetrabenazine for use according to any one of Embodiments 1.1 to 1.16 wherein the movement disorder is selected from tardive dyskinesia, Tourette's syndrome and Huntington's disease.
1.18 (+)-α-Dihydrotetrabenazine for use according to Embodiment 1.17 wherein the movement disorder is Tourette's Syndrome.
1.19 (+)-α-Dihydrotetrabenazine for use according to Embodiment 1.17 wherein the movement disorder is Huntington's Disease.
1.20 (+)-α-Dihydrotetrabenazine for use according to Embodiment 1.17 wherein the movement disorder is tardive dyskinesia.

As described above, the present inventors have found that low dosage regimes of (+)-α-dihydrotetrabenazine are useful in blocking the VMAT2 receptor in the treatment of movement disorders. Accordingly, in further embodiments, the invention provides:
1.21 (+)-α-dihydrotetrabenazine for use as described herein, wherein the treatment comprises administering to the subject an amount of (+)-α-dihydrotetrabenazine between 1 mg and 20 mg per day.
1.22 (+)-α-dihydrotetrabenazine for use as described herein, wherein the treatment comprises administering to the subject an amount of (+)-α-dihydrotetrabenazine between 2 mg and 20 mg per day.
1.23 (+)-α-dihydrotetrabenazine for use as described herein, wherein the treatment comprises administering to the subject an amount of (+)-α-dihydrotetrabenazine 3 mg and 20 mg per day.
1.24 (+)-α-dihydrotetrabenazine for use as described herein, wherein the treatment comprises administering to the subject an amount of (+)-α-dihydrotetrabenazine 2 mg and 15 mg per day.
1.25 (+)-α-dihydrotetrabenazine for use as described herein, wherein the treatment comprises administering to the subject an amount of (+)-α-dihydrotetrabenazine between 3 mg and 15 mg per day.
1.26 (+)-α-dihydrotetrabenazine for use as described herein, wherein the treatment comprises administering to the subject an amount of (+)-α-dihydrotetrabenazine between 5 mg and 15 mg per day.
1.27 (+)-α-dihydrotetrabenazine for use as described herein, wherein the treatment comprises administering to the subject an amount of (+)-α-dihydrotetrabenazine between 5 mg and 10 mg per day.
1.28 (+)-α-dihydrotetrabenazine for use as described herein, wherein the treatment comprises administering to the subject an amount of (+)-α-dihydrotetrabenazine of approximately 5 mg per day.
1.29 (+)-α-dihydrotetrabenazine for use as described herein, wherein the treatment comprises administering to the subject an amount of (+)-α-dihydrotetrabenazine of approximately 7.5 mg per day.
1.30 (+)-α-dihydrotetrabenazine for use as described herein, wherein the treatment comprises administering to the subject an amount of (+)-α-dihydrotetrabenazine of approximately 10 mg per day.
1.31 (+)-α-dihydrotetrabenazine for use as described herein, wherein the treatment comprises administering to the subject an amount of (+)-α-dihydrotetrabenazine of approximately 12.5 mg per day.
1.32 (+)-α-dihydrotetrabenazine for use as described herein, wherein the treatment comprises administering to the subject an amount of (+)-α-dihydrotetrabenazine of approximately 15 mg per day.

In each case, unless the context indicates otherwise, the quantity of (+)-α-dihydrotetrabenazine specified may be administered once per day or in several (e.g. two) doses per day.

In some embodiments, the quantity of (+)-α-dihydrotetrabenazine specified is administered once daily.

The administration of (+)-α-dihydrotetrabenazine typically forms part of a chronic treatment regime. The (+)-α-dihydrotetrabenazine may therefore be administered to a patient for a treatment period of at least a week, more usually at least two weeks, or at least a month, and typically longer than a month. Where a patient is shown to respond well to treatment, the period of treatment can be longer than six months and may extend over a period of years.

The chronic treatment regime may involve the administration of the (+)-α-dihydrotetrabenazine every day, or the treatment regime may include days when no (+)-α-dihydrotetrabenazine is administered.

The dosage administered to the subject may vary during the treatment period. For example, the initial dosage may be increased or decreased depending on the subject's response to the treatment. A subject may, for example, be given an initial low dose to test the subject's tolerance towards the (+)-α-dihydrotetrabenazine, and the dosage thereafter increased as necessary up to the maximum daily intake of 20 mg. Alternatively, an initial daily dosage administered to the patient may be selected so as to give an estimated desired degree of VMAT2 blockage, following which a lower maintenance dose may be given for the remainder of the treatment period, with the option of increasing the dosage should the subject's response to the treatment indicate that an increase is necessary.

The quantity of (+)-α-dihydrotetrabenazine required to achieve the desired therapeutic effect may be dependent on the weight of the subject to be treated. The quantities of (+)-α-dihydrotetrabenazine administered to the subject are expressed in a number of mg/kg, wherein the kg relates the weight of the subject to be treated. The appropriate dosage amount can therefore be calculated by multiplying the mg/kg amount by the weight of the subject to be treated. Accordingly, the invention also provides:
1.37 (+)-α-dihydrotetrabenazine for use as described herein, wherein the treatment comprises administering to the subject between 0.05 mg / kg and 0.3 mg / kg of (+)-α-dihydrotetrabenazine, provided that the total amount of (+)-α-dihydrotetrabenazine administered per day is in the range from 0.5 mg to 20 mg (e.g. 1 mg to 20 mg).
1.38 (+)-α-dihydrotetrabenazine for use as described herein, wherein the treatment comprises administering to the subject between 0.05 mg / kg and 0.2 mg / kg of (+)-α-dihydrotetrabenazine, provided that the total amount of (+)-α-dihydrotetrabenazine administered per day is in the range from 0.5 mg to 20 mg (e.g. 1 mg to 20 mg).
1.39 (+)-α-dihydrotetrabenazine for use as described herein, wherein the treatment comprises administering to the subject between 0.05 mg / kg and 0.1 mg / kg of (+)-α-dihydrotetrabenazine, provided that the total amount of (+)-α-dihydrotetrabenazine administered per day is in the range from 0.5 mg to 20 mg (e.g. 1 mg to 20 mg).

In each of the foregoing aspects and embodiments, the (+)-α-dihydrotetrabenazine can be administered as the free base or as a pharmaceutically acceptable salt. In one embodiment, the (+)-α-dihydrotetrabenazine is administered as a pharmaceutically acceptable salt. In another embodiment, the (+)-α-dihydrotetrabenazine is administered as a free base. The quantities of (+)-α-dihydrotetrabenazine are calculated as the amounts of the free base, or when the (+)-α-dihydrotetrabenazine is in the form of a pharmaceutically acceptable salt, the amount of (+)-α-dihydrotetrabenazine free base present in the pharmaceutically acceptable salt.

The present inventors have found that plasma levels of (+)-α-dihydrotetrabenazine required for effective treatment of hyperkinetic movement disorders can be considerably lower than the plasma levels achieved by administration of Valbenazine as described in WO 2015/171802.

Complete blocking of VMAT2 is considered undesirable as this can lead to unwanted side effects, such as Parkinsonism. The present invention provides plasma levels of (+)-α-dihydrotetrabenazine that are sufficient to give effective treatment of movement disorders but do not block VMAT2 to an extent that causes Parkinsonism and similar side effects. The levels of VMAT2 blocking can be determined by competitive binding studies using Positron Emission Tomography (PET). By co-administering a radioactive ligand with the compound of interest at various concentrations, the proportion of binding sites occupied can be determined (see for example, Matthews et al., "Positron emission tomography molecular imaging for drug development", Br. J. Clin. Pharmacol., 73:2, 175-186). Accordingly, the invention also provides:
1.40 (+)-α-dihydrotetrabenazine for use as described herein, wherein the treatment comprises administering to the subject an amount of (+)-α-dihydrotetrabenazine sufficient to cause a level of blocking of up to 85% of the VMAT2 proteins in the subject.
1.41 (+)-α-dihydrotetrabenazine for use as described herein, wherein the treatment comprises administering to the subject an amount of (+)-α-dihydrotetrabenazine sufficient to cause a level of blocking of up to 80% of the VMAT2 proteins in the subject.
1.42 (+)-α-dihydrotetrabenazine for use as described herein, wherein the treatment comprises administering to the subject an amount of (+)-α-dihydrotetrabenazine sufficient to cause a level of blocking of up to 75% of the VMAT2 proteins in the subject.
1.43 (+)-α-dihydrotetrabenazine for use as described herein, wherein the treatment comprises administering to the subject an amount of (+)-α-dihydrotetrabenazine sufficient to cause a level of blocking of up to 70% of the VMAT2 proteins in the subject.
1.44 (+)-α-dihydrotetrabenazine for use as described herein, wherein the treatment comprises administering to the subject an amount of (+)-α-dihydrotetrabenazine sufficient to cause a level of blocking of from 25% to 85% of the VMAT2 proteins in the subject.
1.45 (+)-α-dihydrotetrabenazine for use as described herein, wherein the treatment comprises administering to the subject an amount of (+)-α-dihydrotetrabenazine sufficient to cause a level of blocking of from 30% to 80% of the VMAT2 proteins in the subject.
1.46 (+)-α-dihydrotetrabenazine for use as described herein, wherein the treatment comprises administering to the subject an amount of (+)-α-dihydrotetrabenazine sufficient to cause a level of blocking of from 35% to 75% of the VMAT2 proteins in the subject.
1.47 (+)-α-dihydrotetrabenazine for use as described herein, wherein the treatment comprises administering to the subject an amount of (+)-α-dihydrotetrabenazine sufficient to cause a level of blocking of from 35% to 70% of the VMAT2 proteins in the subject.
1.48 (+)-α-dihydrotetrabenazine for use as described herein, wherein the treatment comprises administering to the subject an amount of (+)-α-dihydrotetrabenazine sufficient to cause a level of blocking of from 40% to 75% of the VMAT2 proteins in the subject.
1.49 (+)-α-dihydrotetrabenazine for use as described herein, wherein the treatment comprises administering to the subject in need thereof, wherein the method comprises administering to a subject an amount of (+)-α-dihydrotetrabenazine sufficient to cause a level of blocking of from 45% to 75% of the VMAT2 proteins in the subject.
1.50 (+)-α-dihydrotetrabenazine for use as described herein, wherein the treatment comprises administering to the subject in need thereof, wherein the method comprises administering to a subject an amount of (+)-α-dihydrotetrabenazine sufficient to cause a level of blocking of from 35% to 80% of the VMAT2 proteins in the subject.
1.51 (+)-α-dihydrotetrabenazine for use as described herein, wherein the treatment comprises administering to the subject in need thereof, wherein the method comprises administering to a subject an amount of (+)-α-dihydrotetrabenazine sufficient to cause a level of blocking of from 40% to 80% of the VMAT2 proteins in the subject.
1.52 (+)-α-dihydrotetrabenazine for use as described herein, wherein the treatment comprises administering to the subject an amount of (+)-α-dihydrotetrabenazine sufficient to cause a level of blocking of from 45% to 80% of the VMAT2 proteins in the subject.
1.53 (+)-α-dihydrotetrabenazine for use as described herein, wherein the treatment comprises administering to the subject an amount of (+)-α-dihydrotetrabenazine sufficient to cause a level of blocking of from 50% to 80% of the VMAT2 proteins in the subject.
1.54 (+)-α-dihydrotetrabenazine for use as described herein, wherein the treatment comprises administering to the subject an amount of (+)-α-dihydrotetrabenazine sufficient to cause a level of blocking of from 55% to 80% of the VMAT2 proteins in the subject.
1.55 (+)-α-dihydrotetrabenazine for use as described herein, wherein the treatment comprises administering to the subject an amount of (+)-α-dihydrotetrabenazine sufficient to cause a level of blocking of VMAT2 proteins in the subject of between 30% and 70%.
1.56 (+)-α-dihydrotetrabenazine for use as described herein, wherein the treatment comprises administering to the subject in need thereof, wherein the method comprises administering to a subject an amount of (+)-α-dihydrotetrabenazine sufficient to cause a blocking level of VMAT2 proteins in the subject of between 30% and 65%.
1.57 (+)-α-dihydrotetrabenazine for use as described herein, wherein the treatment comprises administering to the subject in need thereof, wherein the method comprises administering to a subject an amount of (+)-α-dihydrotetrabenazine sufficient to cause a blocking level of VMAT2 proteins in the subject of between 30% and 60%.
1.58 (+)-α-dihydrotetrabenazine for use as described herein, wherein the treatment comprises administering to the subject in need thereof, wherein the method comprises administering to a subject an amount of (+)-α-dihydrotetrabenazine sufficient to cause a level blocking of VMAT2 proteins in the subject of between 40% and 80%.
1.59 (+)-α-dihydrotetrabenazine for use as described herein, wherein the treatment comprises administering to the subject in need thereof, wherein the method comprises administering to a subject an amount of (+)-α-dihydrotetrabenazine sufficient to cause a level of blocking of VMAT2 proteins in the subject of between 40% and 75%.
1.60 (+)-α-dihydrotetrabenazine for use as described herein, wherein the treatment comprises administering to the subject in need thereof, wherein the method comprises administering to a subject an amount of (+)-α-dihydrotetrabenazine sufficient to cause a level of blocking of VMAT2 proteins in the subject of between 40% and 70%.
1.61 (+)-α-dihydrotetrabenazine for use as described herein, wherein the treatment comprises administering to the subject in need thereof, wherein the method comprises administering to a subject an amount of (+)-α-dihydrotetrabenazine sufficient to cause a level of blocking of VMAT2 proteins in the subject of between 40% and 65%.
1.62 (+)-α-dihydrotetrabenazine for use as described herein, wherein the treatment comprises administering to the subject in need thereof, wherein the method comprises administering to a subject an amount of (+)-α-dihydrotetrabenazine sufficient to cause a level blocking of VMAT2 proteins in the subject of between 40% and 60%.

In further embodiments of the invention, particular, it is envisaged that low doses of (+)-α-dihydrotetrabenazine may be used in the treatment of hyperkinetic movement disorders in juvenile human subjects aged from 5 years to 16 years old.

In each of Embodiments 1.1 to 1.62, the amounts of (+)-α-dihydrotetrabenazine or a pharmaceutically acceptable salt required to be administered to a subject may depend in part on the speed of metabolism of the compound by the subject.

Metabolism of many drugs (approximately 25% of all current prescription drugs) involves the cytochrome P450 2D6 (CYP2D6) enzyme which is primarily expressed in the liver.

Approximately 7% of the population has a slow acting form of this enzyme and 7% a super-fast acting form. Thirty-five percent are carriers of a non-functional CYP2D6 allele.

As a result, subjects can be classified according to whether they are:
- poor metabolizers - with little or no CYP2D6 function;
- intermediate metabolizers - who metabolize drugs at a rate somewhere between the poor and extensive metabolizers;
- extensive metabolizers - with normal CYP2D6 function; or
- ultrarapid metabolizer - with multiple copies of the CYP2D6 gene are expressed, so greater-than-normal CYP2D6 function occurs.

Thus, considerable variation exists in the efficiency and amount of CYP2D6 enzyme produced between individuals. Hence, for drugs that are metabolized by CYP2D6, certain individuals will eliminate these drugs quickly (ultrarapid metabolizers) while others will do so slowly (poor metabolizers). If a drug is metabolized too quickly, it may decrease the drug's efficacy while if the drug is metabolized too slowly, toxicity may result.

A number of commercially available diagnostic tests are available for determining whether a subject is a fast or slow metabolizer and these include the CYP2D6 Genotyping tests available from Genelex Labs LLC, Seattle, WA 98121, USA, and Trimgen Corporation, Sparks, Maryland 21152, USA.

Therefore, in Embodiments 1.1 to 1.62, the use may include the step of testing a subject to determine the CYP2D6 status of the subject and then using the outcome of the test as a factor in determining the amounts of (+)-α-dihydrotetrabenazine or a pharmaceutically acceptable salt to be administered to the subject. Thus, a fast metabolizer may be administered an amount towards the upper end of a given range whereas a slower metabolizer may be administered a smaller amount.

In each of the foregoing Embodiments 1.1 to 1.62, the unit dosage form (or the substance administered in the method) contains no more than 20% by weight, relative to the (+)-α-dihydrotetrabenazine, of any other isomer of dihydrotetrabenazine; more usually contains no more than 10% by weight, relative to the (+)-α-dihydrotetrabenazine, of any other isomer of dihydrotetrabenazine; preferably contains no more than 5% by weight, relative to the (+)-α-dihydrotetrabenazine, of any other isomer of dihydrotetrabenazine; and more preferably contains no more than 2% by weight, relative to the (+)-α-dihydrotetrabenazine, of any other isomer of dihydrotetrabenazine.

Thus, the (+)-α-dihydrotetrabenazine typically has an isomeric purity of at least 80%.

The term "isomeric purity" in the present context refers to the amount (+)-α-dihydrotetrabenazine present relative to the total amount or concentration of dihydrotetrabenazines of all isomeric forms. For example, if 90% of the total dihydrotetrabenazine present in the composition is (+)-α-dihydrotetrabenazine then the isomeric purity is 90%.

The (+)-α-dihydrotetrabenazine of the invention may have an isomeric purity of greater than 82%, greater than 85%, greater than 87%, greater than 90%, greater than 91%, greater than 92%, greater than 93%, greater than 94%, greater than 95%, greater than 96%, greater than 97%, greater than 98%, greater than 99%, greater than 99.5%, or greater than 99.9%.

### Salts

The dihydrotetrabenazine may be presented as the free base or in the form of salts. All references herein to (+)-α-dihydrotetrabenazine include (+)-α-dihydrotetrabenazine in both free base and salt forms thereof, unless the context indicates otherwise.

In one embodiment (Embodiment 1.63), the (+)-α-dihydrotetrabenazine as defined in any one of Embodiments 1.1 to 1.62 is in the form of the free base.

In another embodiment (Embodiment 1.64), the (+)-α-dihydrotetrabenazine as defined in any one of Embodiments 1.1 to 1.62 is in the form of a salt.

The salts are typically acid addition salts.

The salts can be synthesized from the parent compound by conventional chemical methods such as methods described in Pharmaceutical Salts: Properties, Selection, and Use, P. Heinrich Stahl (Editor), Camille G. Wermuth (Editor), ISBN: 3-90639-026-8, Hardcover, 388 pages, August 2002. Generally, such salts can be prepared by reacting the free base form of the compound with the acid in water or in an organic solvent, or in a mixture of the two; generally, nonaqueous media such as ether, ethyl acetate, ethanol, isopropanol, or acetonitrile are used.

Acid addition salts may be formed with a wide variety of acids, both inorganic and organic. Examples of acid addition salts include salts formed with an acid selected from the group consisting of acetic, 2,2-dichloroacetic, adipic, alginic, ascorbic (e.g. L-ascorbic), L-aspartic, benzenesulphonic, benzoic, 4-acetamidobenzoic, butanoic, (+) camphoric, camphor-sulphonic, (+)-(1*S*)-camphor-10-sulphonic, capric, caproic, caprylic, cinnamic, citric, cyclamic, dodecylsulphuric, ethane-1,2-disulphonic, ethanesulphonic, 2-hydroxyethanesulphonic, formic, fumaric, galactaric, gentisic, glucoheptonic, D-gluconic, glucuronic (e.g. D-glucuronic), glutamic (e.g. L-glutamic), α-oxoglutaric, glycolic, hippuric, hydrobromic, hydrochloric, hydriodic, isethionic, (+)-L-lactic, (±)-DL-lactic, lactobionic, maleic, malic, (-)-L-malic, malonic, (±)-DL-mandelic, methanesulphonic, naphthalene-2-sulphonic, naphthalene-1,5-disulphonic, 1-hydroxy-2-naphthoic, nicotinic, nitric, oleic, orotic, oxalic, palmitic, pamoic, phosphoric, propionic, L-pyroglutamic, salicylic, 4-amino-salicylic, sebacic, stearic, succinic, sulphuric, tannic, (+)-L-tartaric, thiocyanic, p-toluenesulphonic, undecylenic and valeric acids, as well as acylated amino acids and cation exchange resins.

The salt forms of the compound of the invention are typically pharmaceutically acceptable salts, and examples of pharmaceutically acceptable salts are discussed in Berge et al., 1977, "Pharmaceutically Acceptable Salts," J. Pharm. Sci., Vol. 66, pp. 1-19. However, salts that are not pharmaceutically acceptable may also be prepared as intermediate forms which may then be converted into pharmaceutically acceptable salts. Such non-pharmaceutically acceptable salts forms, which may be useful, for example, in the purification or separation of the compounds of the invention, also form part of the invention.

### Isotopes

The (+)-α-dihydrotetrabenazine may contain one or more isotopic substitutions, and a reference to a particular element includes within its scope all isotopes of the element. For example, a reference to hydrogen includes within its scope ¹H, ²H (D), and ³H (T). Similarly, references to carbon and oxygen include within their scope respectively ¹¹C, ¹²C, ¹³C and ¹⁴C and ¹⁶O and ¹⁸O.

Typically, the (+)-α-dihydrotetrabenazine of the invention does not contain isotopes (such as ¹¹C or ³H) in amounts higher than their natural abundance.

In one embodiment, the percentage of the total hydrogen atoms in the (+)-α-dihydrotetrabenazine that are deuterium atoms is less than 2%, more typically less than 1%, more usually less than 0.1%, preferably less than 0.05% and most preferably no more than 0.02%.

In an analogous manner, a reference to a particular functional group also includes within its scope isotopic variations, unless the context indicates otherwise.

The isotopes may be radioactive or non-radioactive. In one embodiment of the invention, the (+)-α-dihydrotetrabenazine contains no radioactive isotopes. Such compounds are preferred for therapeutic use. In another embodiment, however, the (+)-α-dihydrotetrabenazine may contain one or more radioisotopes. Compounds containing such radioisotopes may be useful in a diagnostic context.

### Solvates

### (+)-α-Dihydrotetrabenazine may form solvates.

Examples of solvates are solvates formed by the incorporation into the solid state structure (e.g. crystal structure) of the compounds of the invention of molecules of a non-toxic pharmaceutically acceptable solvent (referred to below as the solvating solvent). Examples of such solvents include water, alcohols (such as ethanol, isopropanol and butanol) and dimethylsulphoxide. Solvates can be prepared by recrystallising the compounds of the invention with a solvent or mixture of solvents containing the solvating solvent. Whether or not a solvate has been formed in any given instance can be determined by subjecting crystals of the compound to analysis using well known and standard techniques such as thermogravimetric analysis (TGE), differential scanning calorimetry (DSC) and X-ray crystallography.

The solvates can be stoichiometric or non-stoichiometric solvates.

Particular solvates are hydrates, and particular examples of hydrates include hemihydrates, monohydrates and dihydrates.

For a more detailed discussion of solvates and the methods used to make and characterise them, see Bryn et al., Solid-State Chemistry of Drugs, Second Edition, published by SSCI, Inc of West Lafayette, IN, USA, 1999, ISBN 0-967-06710-3.

Alternatively, rather than existing as a hydrate, the compound of the invention may be anhydrous. Therefore, in another embodiment, the (+)-α-dihydrotetrabenazine is in an anhydrous form.

### Methods for the Preparation of (+)-α-Dihydrotetrabenazine

(+)-α-Dihydrotetrabenazine (compound of formula (I)) can be prepared from tetrabenazine according to the synthetic route shown in Scheme 1.

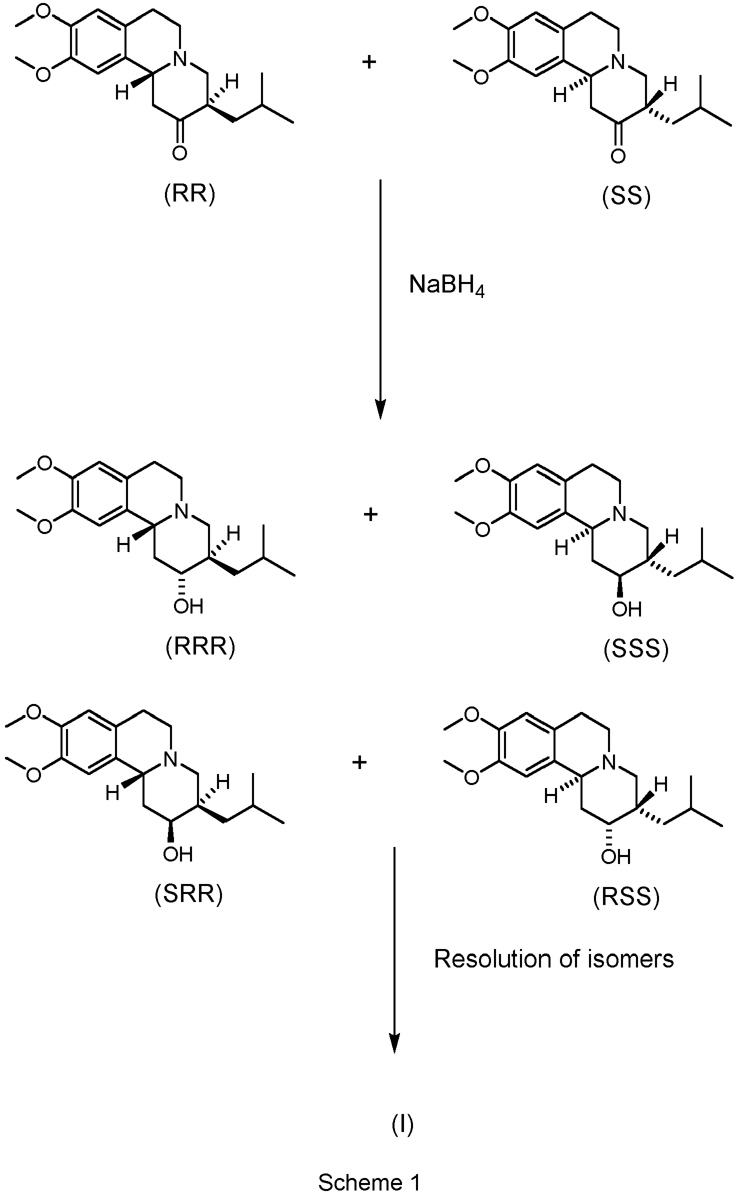

Racemic tetrabenazine (3-isobutyl-9,10-dimethoxy-1,3,4,6,7,11b-hexahydro-2*H-*pyrido[2,1,a]isoquinolin-2-one) containing the *RR* and *SS* isomers of tetrabenazine is reduced with sodium borohydride to afford a mixture of four dihydrotetrabenazine isomers of which a racemic mixture of the α-dihydrotetrabenazines (*RRR* and *SSS* isomers) constitutes the major product and a racemic mixture of the β-dihydrotetrabenazines (the *SRR* and *RSS* isomers) constitutes a minor product. The β-dihydrotetrabenazines can be removed during an initial purification procedure, for example by chromatography or recrystallization and then the racemic α-dihydrotetrabenazines resolved (e.g. by recrystallisation with di-p-toluoyl-L-tartaric acid or (R)-(-)-camphorsulfonic acid or by chiral chromatography), to afford (+)-α-dihydrotetrabenazine (I) ((*2R, 3R, 11bR)*-3-isobutul-9,10-dimethox-1,3,4,6,7,11b-hexahydro-2*H*-pyrido[2,1,a]isoquinolin-2-ol). The stereochemical configuration of (+)-α-dihydrotetrabenazine can be determined, so example by forming a salt such as the mesylate salt in crystalline form and the structure identified by X-ray crystallography.

(+)-α-Dihydrotetrabenazine can also be prepared according to Yao et al., "Preparation and evaluation of tetrabenazine enantiomers and all eight stereoisomers of dihydrotetrabenazine as VMAT2 inhibitors", Eur. J. Med. Chem., (2011), 46, pp. 1841 - 1848.

### Pharmaceutical Formulations

In each of Embodiments 1.1 to 1.64, where the compound is administered as a pharmaceutical unit dosage form, or other pharmaceutical composition, the pharmaceutical compositions of the invention can be in any form suitable for oral, parenteral, topical, intranasal, intrabronchial, ophthalmic, otic, rectal, intra-vaginal, or transdermal administration. Where the compositions are intended for parenteral administration, they can be formulated for intravenous, intramuscular, intraperitoneal, subcutaneous administration or for direct delivery into a target organ or tissue by injection, infusion or other means of delivery.

Pharmaceutical dosage forms suitable for oral administration include tablets, capsules, caplets, pills, lozenges, syrups, solutions, sprays, powders, granules, elixirs and suspensions, sublingual tablets, sprays, wafers or patches and buccal patches. In one embodiment, the dosage form is a tablet. In another embodiment, the dosage form is a capsule.

A particular group of pharmaceutical dosage forms for use in accordance with Embodiments 1.1 to 1.64 consists of tablets, capsules, solutions, syrups, suspensions and gels.

Pharmaceutical compositions containing the dihydrotetrabenazine compound of the invention can be formulated in accordance with known techniques, see for example, Remington's Pharmaceutical Sciences, Mack Publishing Company, Easton, PA, USA.

Thus, tablet compositions can contain a unit dosage of active compound together with an inert diluent or carrier such as a sugar or sugar alcohol, e.g.; lactose, sucrose, sorbitol or mannitol; and/or a non-sugar derived diluent such as sodium carbonate, calcium phosphate, talc, calcium carbonate, or a cellulose or derivative thereof such as methyl cellulose, ethyl cellulose, hydroxypropyl methyl cellulose, and starches such as corn starch. Tablets may also contain such standard ingredients as binding and granulating agents such as polyvinylpyrrolidone, disintegrants (e.g. swellable crosslinked polymers such as crosslinked carboxymethylcellulose), lubricating agents (e.g. stearates), preservatives (e.g. parabens), antioxidants (e.g. BHT), buffering agents (for example phosphate or citrate buffers), and effervescent agents such as citrate/bicarbonate mixtures. Such excipients are well known and do not need to be discussed in detail here.

Capsule formulations may be of the hard gelatin or soft gelatin variety and can contain the active component in solid, semi-solid, or liquid form. Gelatin capsules can be formed from animal gelatin or synthetic or plant derived equivalents thereof.

The solid dosage forms (e.g.; tablets, capsules etc.) can be coated or un-coated, but typically have a coating, for example a protective film coating (e.g. a wax or varnish) or a release controlling coating. The coating (e.g. a Eudragit ^{™} type polymer) can be designed to release (+)-α-dihydrotetrabenazine at a desired location within the gastro-intestinal tract. Thus, the coating can be selected so as to degrade under certain pH conditions within the gastrointestinal tract, thereby selectively release the compound in the stomach or in the ileum or duodenum.

The release controlling coating can be designed to release (+)-α-dihydrotetrabenazine at such as rate that a therapeutically effective plasma concentration is maintained for at least 2 hours, more typically at least 3 hours, for example at least 4 hours, or at least 5 hours.

The composition may comprise a multiplicity of individual units such as pellets or minitablets which each contain (+)-α-dihydrotetrabenazine and which may each be coated with a release controlling agent.

Instead of, or in addition to, a coating, the drug can be presented in a solid matrix comprising a release controlling agent, for example a release delaying agent which may be adapted to selectively release the compound under conditions of varying acidity or alkalinity in the gastrointestinal tract. Alternatively, the matrix material or release retarding coating can take the form of an erodible polymer (e.g. a maleic anhydride polymer) which is substantially continuously eroded as the dosage form passes through the gastrointestinal tract.

Compositions for topical use include ointments, creams, sprays, patches, gels, liquid drops and inserts (for example intraocular inserts). Such compositions can be formulated in accordance with known methods.

Compositions for parenteral administration are typically presented as sterile aqueous or oily solutions or fine suspensions, or may be provided in finely divided sterile powder form for making up extemporaneously with sterile water for injection.

Examples of formulations for rectal or intra-vaginal administration include pessaries and suppositories which may be, for example, formed from a shaped mouldable or waxy material containing the active compound.

Compositions for administration by inhalation may take the form of inhalable powder compositions or liquid or powder sprays, and can be administrated in standard form using powder inhaler devices or aerosol dispensing devices. Such devices are well known. For administration by inhalation, the powdered formulations typically comprise the active compound together with an inert solid powdered diluent such as lactose.

Particular pharmaceutical compositions of the invention are compositions selected from:
- Sublingual compositions;
- Intranasal;
- Pellets or tablets formulated to provide release kinetics corresponding to zero order release of the active compound;
- Pellets or tablets formulated to provide first fast release followed by constant rate release (zero order) of the active compound;
- Pellets or tablets formulated to provide a mixture of first order and zero order release of the active compound; and
- Pellets or tablets formulated to provide a combination of zero order and first order release of the active compound; and optionally a further order of release of the active compound selected from second, third and fourth orders of release and combinations thereof.

Pellets and tablets formulated to provide release kinetics of the types defined above can be prepared according to methods well known the skilled person; for example as described in Remington's Pharmaceutical Sciences (idem) and "Remington - The Science and Practice of Pharmacy, 21st edition, 2006, ISBN 0-7817-4673-6.

The compounds of the inventions will generally be presented in unit dosage form and, as such, will typically contain sufficient compound to provide a desired level of biological activity. Such amounts are set out above.

The active compound will be administered to a subject (patient) in need thereof (for example a human or animal patient) in an amount sufficient to achieve the desired therapeutic effect.

### Brief Description of the Drawings

Figure 1 is a plot of % VMAT2 binding vs body weight after administration of doses of 7.5 mg, 15 mg and 22.5 mg of (+)-α-dihydrotetrabenazine to human subjects.
Figure 2 is a plot of % VMAT2 binding vs amounts of (+)-α-dihydrotetrabenazine administered to human subjects in mg/kg body weight.
Figure 3 shows the average total distance travelled by rats when treated with vehicle (with or without amphetamine induction) and (+)-α-dihydrotetrabenazine at doses of 0.5, 1, 1.5 and 2 mg/kg and risperidone at a dose of 1 mg/kg in amphetamine-induced rats, as described in Example 2, Study 1 below.
Figure 4 shows the average total stereotypic behaviour by rats when treated with vehicle (with or without amphetamine induction) and (+)-α-dihydrotetrabenazine at doses of 0.5, 1, 1.5 and 2 mg/kg and risperidone at a dose of 1 mg/kg in amphetamine-induced rats, as described in Example 2, Study 1 below.
Figure 5 shows the average total distance travelled by rats when treated with vehicle (with or without amphetamine induction) and (+)-α-dihydrotetrabenazine at doses of 0.1 mg/kg and 0.25 mg/kg and risperidone at a dose of 1 mg/kg in amphetamine-induced rats, as described in Example 2, Study 2 below.
Figure 6 shows the average total stereotypic behaviour by rats when treated with vehicle (with or without amphetamine induction) and (+)-α-dihydrotetrabenazine at doses of 0.1 mg/kg and 0.25 mg/kg and risperidone at a dose of 1 mg/kg in amphetamine-induced rats, as described in Example 2, Study 2 below.
Figure 7 shows the average total distance travelled by rats when treated with vehicle and (+)-α-dihydrotetrabenazine at a dose of 2.5 mg/kg or 5 mg/kg and risperidone at a dose of 1 mg/kg in rats without amphetamine induction, as described in Example 2, Study 3 below.
Figure 8 shows the average total stereotypic behaviour by rats when treated with vehicle and (+)-α-dihydrotetrabenazine at a dose of 2.5 mg/kg or 5 mg/kg and risperidone at a dose of 1 mg/kg in rats without amphetamine induction, as described in Example 2, Study 3 below.
Figure 9 shows the average total distance travelled by rats when treated with vehicle and (+)-α-dihydrotetrabenazine and Valbenazine each at a dose of 1 mg/kg or 1.5 mg/kg and risperidone at a dose of 1 mg/kg in amphetamine-induced rats, as described in Example 2, Study 4 below.
Figure 10 shows the average total stereotypic behaviour by rats when treated with vehicle and (+)-α-dihydrotetrabenazine and Valbenazine each at a dose of 1 mg/kg or 1.5 mg/kg and risperidone at a dose of 1 mg/kg in amphetamine-induced rats, as described in Example 2, Study 4 below.

### EXAMPLES

The following non-limiting examples illustrate the synthesis and properties of the (+)-alpha-dihydrotetrabenazine salt of the invention.

### EXAMPLE 1

(+)-α-Dihydrotetrabenazine in defined amounts was administered by oral dosing to five human volunteers. In four of the volunteers, blood sample were taken at 30, 60, 120 and 180 minutes after drug administration. Blood samples were not taken from the fifth volunteer. At 60 minutes after drug administration, PET scans were initiated and these were stopped at 120 minutes after drug administration.

The experiment was carried out at dosages of 7.5 mg, 15 mg and 22.5 mg.

### RESULTS

Table 1 shows the plasma concentrations in nanogrammes/ml of (+)-α-dihydrotetrabenazine in five human subjects, 0.5, 1, 1.5, 2 and 3 hours after a dose of 7.5 mg, 15 mg and 22.5 mg. Table 2 shows the % VMAT2 blocking following administration of 7.5 mg, 15 mg and 22.5 mg of (+)-α-dihydrotetrabenazine in all five subjects.

**Table 1**

| | | **Subject** # | | | | | |
|---|---|---|---|---|---|---|---|
| | | **1** | **2** | **3** | **4** | **5** | **6** |
| **Body Weight (kg)** | | 112 | 76 | 129 | 59 | 91 | 68 |

| **Dose (oral)** | **Time (h)** | | | | | | |
|---|---|---|---|---|---|---|---|
| 7.5 mg | | | | | | | |
| | 0.5 | BLQ | 0.531 | 0.216 | 8.43 | 6.68 | BLQ |
| | 1 | 0.94 | 13.7 | 4.35 | 15.0 | 9.8 | 3.77 |
| | 1.5 | 2.39 | 10.8 | 6.91 | 20.7 | 13.5 | 3.06 |
| | 2 | 2.44 | 14.0 | 5.03 | 17.6 | 10.7 | 4.33 |
| | 3 | 3.01 | 22.2 | 6.96 | 19.6 | 11.2 | 9.18 |
| 15 mg | | | | | | | |
| | 0.5 | 4.02 | 7.99 | 1.2 | 26.7 | 15.6 | 5.41 |
| | 1 | 11.1 | 22.8 | 14.3 | 53.8 | 34.2 | 10.6 |
| | 1.5 | 10.7 | 46.4 | 17.9 | 42.5 | 27.1 | 16.1 |
| | 2 | 10.2 | 35.7 | 12.0 | 53.3 | 31.8 | 17.4 |
| | 3 | 10.6 | 46.5 | 18.2 | 60.2 | 27.1 | 14.9 |
| | | | | | | | |
| 22.5 mg | | | | | | | |
| | 0.5 | 9.61 | 5.23 | 9.04 | ND | 55.3 | 5.58 |
| | 1 | 18.0 | 21.8 | 34.7 | ND | 45.1 | 8.01 |
| | 1.5 | 16.8 | 36.2 | 29.8 | ND | 46.2 | 5.87 |
| | 2 | 14.9 | 40.2 | 26.3 | ND | 41.9 | 12.5 |
| | 3 | 13.2 | 51.8 | 17.3 | ND | 42.5 | 18.7 |
| *BLQ - Below level of quantitation, ND - Not done* | | | | | | | |

**Table 2**

| | | **Subject #** | | | | | |
|---|---|---|---|---|---|---|---|
| | | **1** | **2** | **3** | **4** | **5** | **6** |
| **Body Weight (kg)** | | 112 | 76 | 129 | 59 | 91 | 68 |
| **Dose (oral)** | 7.5 mg | 54 | 73 | 62 | 84 | 73 | 60 |
| | 15 mg | 73 | 83 | 69 | 89 | 79 | 76 |
| | 22.5 mg | 75 | 82 | 74 | ND | 82 | 62 |

Although in subjects with a lower body weight, higher (+)-α-dihydrotetrabenazine blood plasma concentrations were observed for a given dose, it can be seen that even in heavier individuals, at least 50% % VMAT2 blocking was observed at doses as low as 7.5 mg and, in lighter individuals, significantly higher % VMAT2 binding was. It was also observed that during the period of PET scanning, average plasma levels of less than 15 ng/ml gave rise to % VMAT2 binding of at least 50%.

The data demonstrate that very low doses of (+)-α-dihydrotetrabenazine resulting in plasma concentrations of less than 15 ng/ml can still give high levels of VMAT2 blocking.

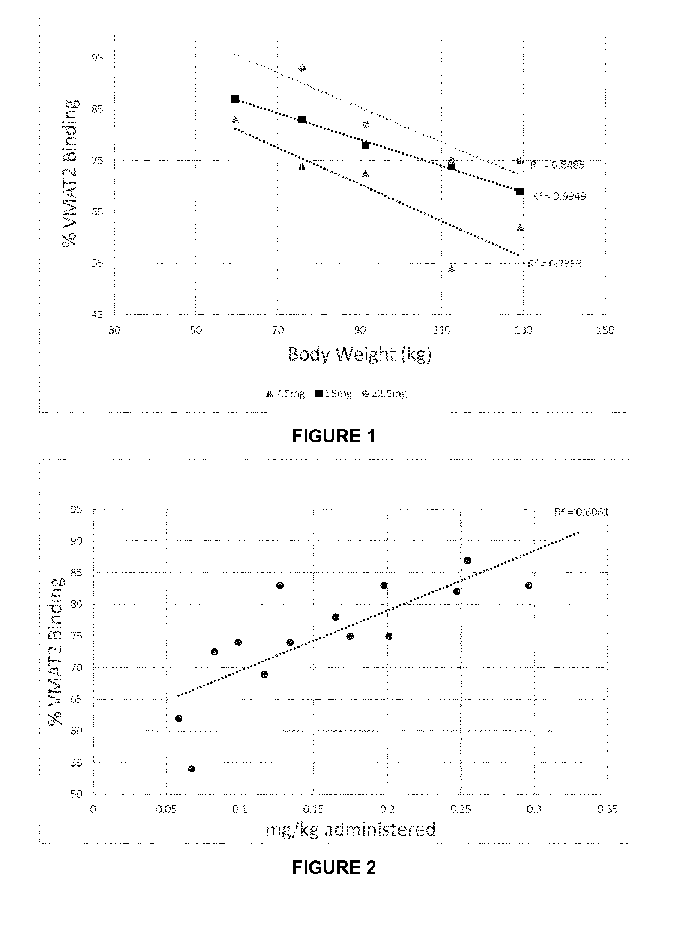

The data for subjects 1 to 5 were used to prepare plots (Figure 1) of % VMAT2 binding against body weight at the three dosage levels and plots (Figure 2) of % VMAt2 binding against the amount (in mg/kg body weight) of (+)-α-dihydrotetrabenazine administered. The data for subject 6, which are somewhat anomalous, were not included in Figure 1 or Figure 2.

Figure 1 shows the % VMAT2 binding against body weight for each dose of (+)-α-DHTBZ administered (7.5 mg, 15 mg and 22.5 mg) based on the data above. As can be seen, for a given dose there is a good correlation between body weight and %VMAT2 binding.

Figure 2 shows the % VMAT2 binding against the amount of (+)-α-DHTBZ per kg of the body weight of the subject based on the data above. Again it can be seen that there is a good correlation between amount of (+)-α-DHTBZ per weight of the subject (mg/kg value) and %VMAT2 binding.

Therefore, it is expected that the amount of (+)-α-DHTBZ that needs to be administered to provide a given VMAT2 binding level will depend greatly on the weight of the subject.

### EXAMPLE 2 - Comparison of the effect of dihydrotetrabenazines and risperidone on amphetamine-induced hyperlocomotion

Dopaminergic models for Tourette's syndrome use systemic or focal administration of dopamine agonists such as amphetamine. After injection with amphetamine, a test animal expresses stereotypic behaviour. In particular, involvement of a dopaminergic system implicated in Tourette's syndrome wild type mice and rats can be stimulated with amphetamine and the resulting hyperactivity and stereotypies can be reversed with dopamine antagonists such as risperidone and haloperidol (Tourette's syndrome - Animal Models for Screening, Charles River Discovery Research Services, Finland).

Amphetamine produced a rise in extracellular concentrations of brain dopamine and concomitant behavioural manifestations in the rat and other species. At relatively low doses (1.2 ng/kg i.p.) amphetamine increases locomotor behaviour, ceases movement and gives way to a stationary posture accompanied by highly repetitive rapid head movements. This latter non-locomotor phase of stimulation is referred to as focused stereotypy. The stereotypy can last for over an hour and is usually followed by a period of locomotor stimulation (Schiorring 1971).

Administration of dopamine agonists (such as amphetamine) is known to induce behavioural stereotypies and sensorimotor gating disruption. Also, dopaminergic, cholinergic (TANs) and HDC models (subsequent to stress and/or amphetamine injection) are known to show an increase in stereotypic behaviours (Yaol et al 2016).

Amphetamine induced stereotype behaviour has also been evaluated as a model for the movement disorder condition, tardive dyskinesia (see Rubovitis et al (1972)).

The atypical antipsychotic drug risperidone is commonly used for the treatment of Tourette's syndrome and has been described (J. D. Walkup, A Guide to Tourette Syndrome Medications, Publ. 2008, The National Tourette Syndrome Association, Inc.) as being probably the best atypical antipsychotic for tic suppression with potentially less risk of motor side effects than haloperidol and fluphenazine.

Three studies were carried out to compare the effects of dihydrotetrabenazines and risperidone on amphetamine-induced and non-amphetamine-induced hyperlocomotion in rats, on the basis that, for the reasons given above, locomotor studies are useful models for Tourette's syndrome and other movement disorders.

### MATERIALS AND METHODS

### Equipment

Open field arena, Med Associates Inc.
Plastic syringes 1 ml, Terumo. Ref: SS-01T1
Animal feeding needle 15 G, Instech Solomon, Cat: 72-4446
Sartorius Mechatronics Scale A22101, Sartorius Weighting Technology, Germany
Needle 27 G Terumo Myjector, 0,5 ml, Ref: 8300010463
Plastic syringes 3 ml, Soft-Ject, Ref: 8300005761
BD Microtainer K2EDTA tubes Ref: 365975
Matrix 0,75 ml, Alphanum Tubes, Thermo Scientific, Ref: 4274
Microplate Devices, Uniplate 24 wells, 10 ml, Ref: 734-1217
Thermo Electron Corp. Heraeus Fresco 17, refrigerated centrifuge

### Test Animals

All animal experiments were carried out according to the National Institute of Health (NIH) guidelines for the care and use of laboratory animals, and approved by the National Animal Experiment Board, Finland. Male CD (Charles River Laboratories, Germany) at weight range of 200-250 g (165-200 g upon arrival) were used for the experiments. Animals were housed at a standard temperature (22 ± 1°C) and in a light-controlled environment (lights on from 7 am to 8 pm) with ad libitum access to food and water.

### Methods

Locomotor activity of the rats was tested in open field arena. The open field test was performed during the rat light cycle and under a normal lighting evenly distributed to the test chambers. The paths of the rats were recorded by activity monitor (Med. Associates Inc.).

Dosing the vehicle, vehicle-amphetamine, (+)-α-DHTBZ or risperidone was done prior to LMA test. The rats were placed in the centre of the arena, and the path was recorded for 60 minutes.

### Endpoint, Blood Samples and Tissue Processing

Within 10 minutes from the end of the test animals were euthanized by an overdose of CO₂. The terminal blood sample was collected with cardiac puncture from all compound treated rats from each group excluding vehicle rats. 0.5 ml of blood was collected with syringe attached to 18 G needle and moved into precooled K2-EDTA microtubes. The EDTA microtube was inverted several times to mix up the EDTA and blood. Tubes were then immediately put on wet ice and centrifuged (Heraeus Fresco 17) within 10-15 minutes of collecting (9.6 x1000 G/ 10 x 1000 RPM, +4°C for 2 min), and 200 µl of plasma was collected in 96-tube plates (Matrix Technologies ScreenMates 0.75 ml Alphanumeric Round-Bottom Storage tubes, PP) on dry ice according to sample map.

After collection of blood the neck was dislocated at the base of the skull. Brain was collected and weighed. Brain weights were recorded and the brain was frozen on dry ice on the 24 well plate.

The plasma and brain samples were stored at -80°C until sent for analysis or destroyed.

### STUDY 1

The effects on stereotypic behaviour and the distance travelled in rats following administration of (+)-α-dihydrotetrabenazine dosed at 0.5 mg/kg to 2 mg/kg, as well as risperidone at 1 mg/kg, were studied.

Animals were grouped as follows:
- Group 1: 10 rats treated with Vehicle (t= 0 min) and Vehicle (t= 30 min)
- Group 2: 10 rats treated with Vehicle (t= 0 min) and Amphetamine (t= 30 min)
- Group 3: 10 rats treated with (+)-α-DHTBZ 0.5 mg/kg (t=0 min) and Amphetamine (t= 30 min)
- Group 4: 10 rats treated with (+)-α-DHTBZ 1 mg/kg (t=0 min) and Amphetamine (t= 30 min)
- Group 5: 10 rats treated with (+)-α-DHTBZ 1.5 mg/kg (t=0 min) and Amphetamine (t= 30 min)
- Group 6: 10 rats treated with (+)-α-DHTBZ 2 mg/kg (t=0 min) and Amphetamine (t= 30 min)
- Group 7: 10 rats treated with risperidone 1 mg/kg (t=0 min) and Amphetamine (t= 30 min)

### Results

### 1. Distance Travelled

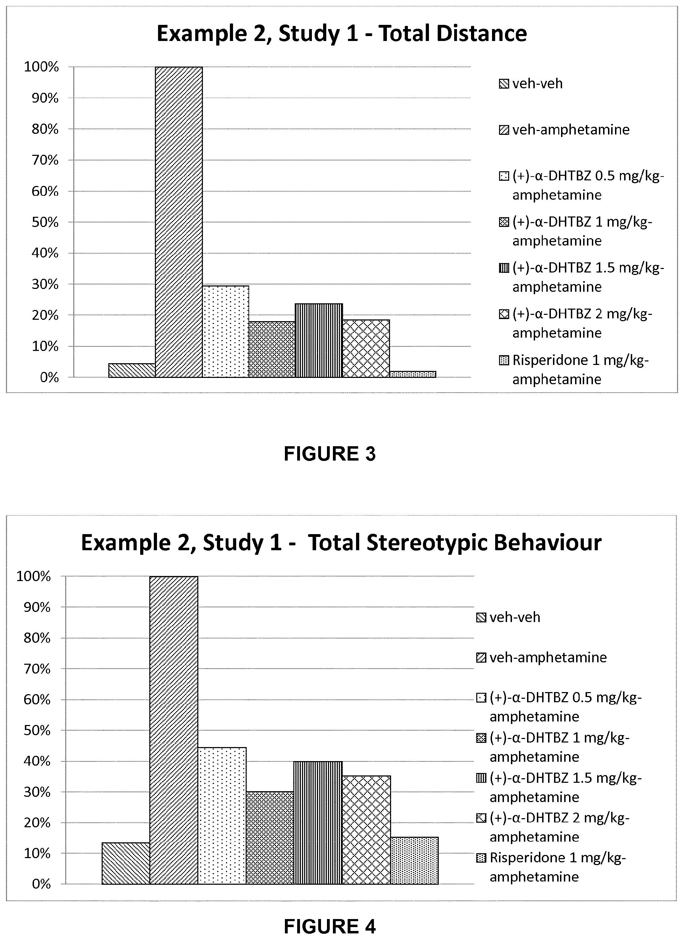

Rats dosed with either vehicle, (+)-α-DHTBZ 0.5 mg/kg, (+)-α-DHTBZ 1 mg/kg, (+)-α-DHTBZ 1.5 mg/kg, (+)-α-DHTBZ 2 mg/kg or Risperidone 1 mg/kg were subjected to LMA testing first for 30 min and then for 60 minutes after vehicle or amphetamine challenge. Resulting locomotor activity was evaluated in 3 min bins and as a total over the testing period. The normalised total distance travelled over the testing time is presented in Figure 3.

When compared to the vehicle-vehicle group the vehicle-amphetamine was significantly different. When compared to vehicle-amphetamine group the vehicle-vehicle, (+)-α-DHTBZ 0.5 mg/kg, (+)-α-DHTBZ 1 mg/kg, (+)-α-DHTBZ 1.5 mg/kg, (+)-α-DHTBZ 2 mg/kg and risperidone 1 mg/kg were significantly different.

### 2. Stereotypic Behaviour

Rats dosed with either vehicle, (+)-α-DHTBZ 0.5 mg/kg, (+)-α-DHTBZ 1 mg/kg, (+)-α-DHTBZ 1.5 mg/kg, (+)-α-DHTBZ 2 mg/kg or Risperidone 1 mg/kg were subjected to LMA testing first for 30 min and then for 60 minutes after vehicle or amphetamine challenge. Resulting stereotypic activity was evaluated in 3 min bins and as a total over the testing period. The normalised total stereotypic behaviour over the testing time is presented in Figure 4.

When compared to the vehicle-vehicle group the vehicle-amphetamine, (+)-α-DHTBZ 0.5 mg/kg and (+)-α-DHTBZ 1.5 mg/kg were significantly different. When compared to vehicle-amphetamine group the vehicle-vehicle, (+)-α-DHTBZ 0.5 mg/kg, (+)-α-DHTBZ 1 mg/kg, (+)-α-DHTBZ 1.5 mg/kg, (+)-α-DHTBZ 2 mg/kg and risperidone 1 mg/kg were significantly different.

### Conclusions

This study evaluated the effect of (+)-α-DHTBZ at doses 0.5 mg/kg, 1 mg/kg, 1.5 mg/kg and 2 mg/kg and risperidone at dose 1 mg/kg on amphetamine induced locomotor activity in male CD rats.

(+)-α-DHTBZ at all the tested doses and risperidone 1 mg/kg led to lower locomotor activity when compared to the vehicle-amphetamine group. (+)-α-DHTBZ at all the tested doses and risperidone 1 mg/kg led to reduced stereotypic behaviour when compared to the vehicle-amphetamine group. Both of the measured parameters suggest that (+)-α-DHTBZ has a sedative effect similar to risperidone.

### STUDY 2

The effects on stereotypic behaviour and the distance travelled in rats following administration of (+)-α-dihydrotetrabenazine dosed at 0.1 mg/kg to 0.25 mg/kg, as well as risperidone at 1 mg/kg, were studied.

Animals were grouped as follows:
- Group 1: 10 rats treated with Vehicle (t= 0 min) and Vehicle (t= 30 min)
- Group 2: 10 rats treated with Vehicle (t= 0 min) and Amphetamine (t= 30 min)
- Group 3: 10 rats treated with (+)-α-DHTBZ 0.1 mg/kg (t=0 min) and Amphetamine (t=30 min)
- Group 4: 10 rats treated with (+)-α-DHTBZ 0.25 mg/kg (t=0 min) and Amphetamine (t=30 min)
- Group 5: 10 rats treated with risperidone 1 mg/kg (t=0 min) and Amphetamine (t= 30 min)

### Results

### 1 Distance Travelled

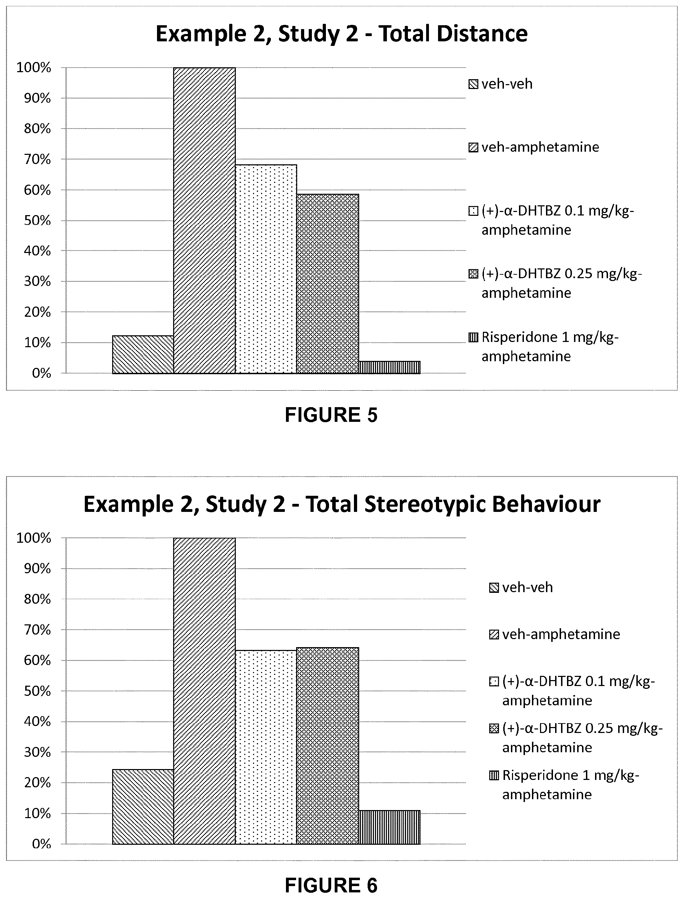

Rats dosed with either vehicle, (+)-α-DHTBZ 0.1 mg/kg, (+)-α-DHTBZ 0.25 mg/kg, or Risperidone 1 mg/kg were subjected to LMA testing first for 30 min and then for 60 minutes after vehicle or amphetamine challenge. Resulting locomotor activity was evaluated in 3 min bins and as a total over the testing period. The normalised total distance travelled over the testing time is presented in Figure 5.

When compared to vehicle-amphetamine group the vehicle-vehicle, (+)-α-DHTBZ 0.25 mg/kg and risperidone 1 mg/kg were significantly different.

### 2 Stereotypic Behaviour

Rats dosed with either vehicle, (+)-α-DHTBZ 0.1 mg/kg, (+)-α-DHTBZ 0.25 mg/kg, or Risperidone 1 mg/kg were subjected to LMA testing first for 30 min and then for 60 minutes after vehicle or amphetamine challenge. Resulting stereotypic activity was evaluated in 3 min bins and as a total over the testing period. The normalised total stereotypic behaviour over the testing time is presented in Figure 6.

When compared to vehicle-amphetamine group the vehicle-vehicle, (+)-α-DHTBZ 0.1 mg/kg, (+)-α-DHTBZ 0.25 mg/kg and risperidone 1 mg/kg were significantly different.

### Conclusions

This study evaluated the effect of (+)-α-DHTBZ at doses of 0.1 mg/kg and 0.25 mg/kg and risperidone at dose 1 mg/kg on amphetamine induced locomotor activity in male CD rats.

(+)-α-DHTBZ at 0.25 mg/kg and risperidone 1 mg/kg led to lower locomotor activity when compared to the vehicle-amphetamine group. (+)-α-DHTBZ at both the tested doses and risperidone 1 mg/kg led to reduced stereotypic behaviour when compared to the vehicle-amphetamine group.

### STUDY 3

The effects of (+)-α-dihydrotetrabenazine and risperidone on in non-amphetamine induced rats was studied. Animals were grouped as follows:
- Group 1: 10 rats treated with Vehicle
- Group 2: 10 rats treated with (+)-α-DHTBZ 2.5 mg/kg
- Group 3: 10 rats treated with (+)-α-DHTBZ 5 mg/kg
- Group 4: 10 rats treated with risperidone 1 mg/kg

### Results

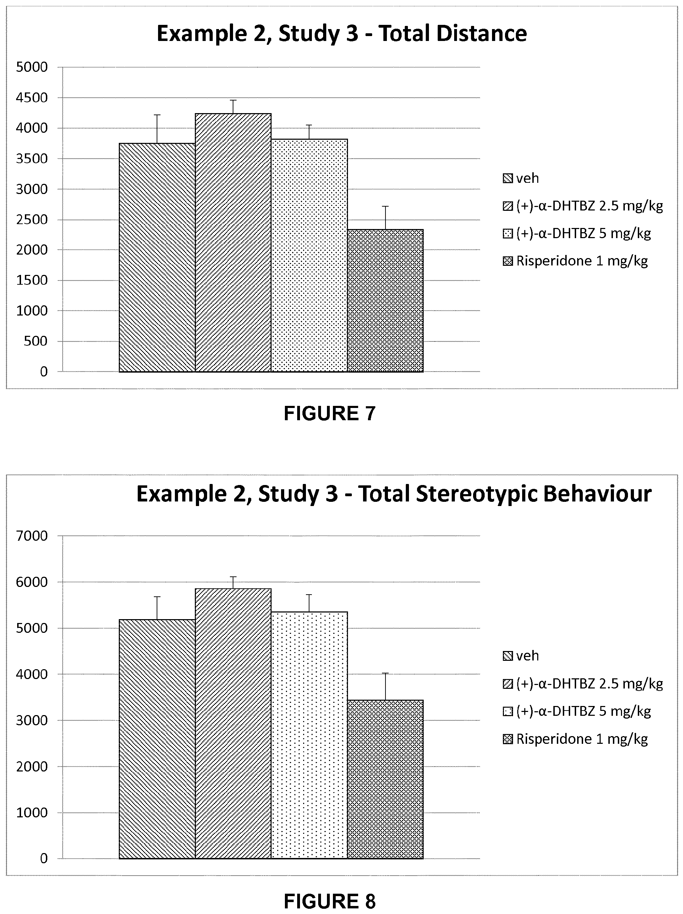

In non-induced rats, the total movement and stereotypic behaviour in rats treated with the vehicle were comparable to (+)-α-dihydrotetrabenazine (see Figures 7 and 8). However, rats treated with risperidone had reduced total movement and reduced total stereotypic behaviour.

### STUDY 4

The effects on stereotypic behaviour and the distance travelled in rats following administration of (+)-α-dihydrotetrabenazine and Valbenazine both dosed at 1 mg/kg and 1.5 mg/kg, as well as risperidone at 1 mg/kg, were studied.

Animals were grouped as follows:
- Group 1: 10 rats treated with Vehicle (t= 0 min) and Vehicle (t= 30 min)
- Group 2: 10 rats treated with Vehicle (t= 0 min) and Amphetamine (t= 30 min)
- Group 3: 10 rats treated with (+)-α-DHTBZ 1 mg/kg (t=0 min) and Amphetamine (t=30 min)
- Group 4: 10 rats treated with (+)-α-DHTBZ 1.5 mg/kg (t=0 min) and Amphetamine (t=30 min)
- Group 5: 10 rats treated with Valbenazine 1 mg/kg (t=0 min) and Amphetamine (t=30 min)
- Group 6: 10 rats treated with Valbenazine 1.5 mg/kg (t=0 min) and Amphetamine (t=30 min)
- Group 7: 10 rats treated with risperidone 1 mg/kg (t=0 min) and Amphetamine (t= 30 min)

### Results

### 1 Distance Travelled

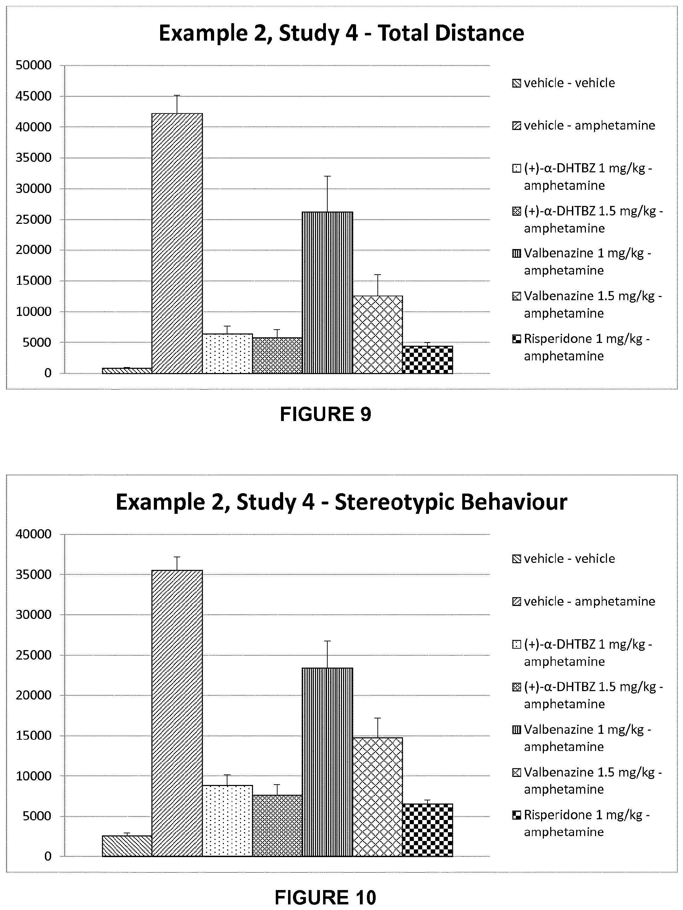

Rats dosed with either vehicle, (+)-α-DHTBZ, Valbenazine or Risperidone were subjected to LMA testing first for 30 min and then for 60 minutes after vehicle or amphetamine challenge. Resulting locomotor activity was evaluated in 3 min bins and as a total over the testing period. The total distance travelled over the testing time is presented in Figure 9.

When compared to vehicle-amphetamine group the vehicle-vehicle, (+)-α-DHTBZ (at 1 mg/kg and 1.5 mg/kg), Valbenazine (at 1 mg/kg and 1.5 mg/kg) and risperidone 1 mg/kg were significantly different.

### 2 Stereotypic Behaviour

Rats dosed with either vehicle, (+)-α-DHTBZ 0.1 mg/kg, (+)-α-DHTBZ 0.25 mg/kg, or Risperidone 1 mg/kg were subjected to LMA testing first for 30 min and then for 60 minutes after vehicle or amphetamine challenge. Resulting stereotypic activity was evaluated in 3 min bins and as a total over the testing period. The normalised total stereotypic behaviour over the testing time is presented in Figure 10.

When compared to vehicle-amphetamine group the vehicle-vehicle, (+)-α-DHTBZ (at 1 mg/kg and 1.5 mg/kg), Valbenazine (at 1 mg/kg and 1.5 mg/kg) and risperidone 1 mg/kg were significantly different.

### Conclusions

This study evaluated the effect of (+)-α-DHTBZ and Valbenazine both at doses of 1 mg/kg and 1.5 mg/kg and risperidone at dose 1 mg/kg on amphetamine induced locomotor activity in male CD rats.

(+)-α-DHTBZ at 1 mg/kg and 1.5 mg/kg led to lower locomotor activity when compared to the vehicle-amphetamine group and the corresponding dose of Valbenazine. (+)-α-DHTBZ at both the tested doses led to reduced stereotypic behaviour when compared to the vehicle-amphetamine group and the corresponding dose of Valbenazine.

### Comments

Studies 1 and 2 in Example 2 show the effectiveness of doses of (+)-α-dihydrotetrabenazine as low as 0.1 mg/kg in reducing movement in amphetamine-induced rats. It is therefore expected that such low dose regimes may also be useful in treating hyperkinetic movement disorders in humans.

Study 3 in Example 2 suggests that following administration of low doses of (+)-α-dihydrotetrabenazine whereas abnormal movements of the type found in movement disorders will be reduced or suppressed by the drug, normal movements will not be. This is in contrast to risperidone, a well-used treatment for movement disorders, where the levels of both normal and abnormal movements can be reduced by administration of the drug.

Study 4 in Example 2 shows the increased effectiveness of (+)-α-dihydrotetrabenazine over Valbenazine.

## Claims

1. (+)-α-Dihydrotetrabenazine or a pharmaceutically acceptable salt thereof for use in treating a hyperkinetic movement disorder wherein the treatment comprises administering to a subject an effective amount of (+)-α-dihydrotetrabenazine or a pharmaceutical salt thereof, wherein the said effective amount corresponds to an amount of (+)-α-dihydrotetrabenazine free base of from 0.05 mg/kg to 0.3 mg/kg per day.

2. (+)-α-Dihydrotetrabenazine or a pharmaceutically acceptable salt thereof for use according to claim 1, provided that the total amount of (+)-α-dihydrotetrabenazine administered per day is in the range from 0.5 mg to 20mg.

3. (+)-α-Dihydrotetrabenazine or a pharmaceutically acceptable salt thereof for use according to claim 1 or claim 2 wherein the hyperkinetic movement disorder is selected from tardive dyskinesia, Tourette's syndrome and Huntington's disease.

4. (+)-α-Dihydrotetrabenazine or a pharmaceutically acceptable salt thereof for use according to claim 3 wherein the hyperkinetic movement disorder is Tourette's Syndrome.

5. (+)-α-Dihydrotetrabenazine or a pharmaceutically acceptable salt thereof for use according to claim 3 wherein the hyperkinetic movement disorder is Huntington's Disease.

6. (+)-α-Dihydrotetrabenazine or a pharmaceutically acceptable salt thereof for use according to claim 3 wherein the hyperkinetic movement disorder is tardive dyskinesia.

7. (+)-α-Dihydrotetrabenazine or a pharmaceutically acceptable salt thereof for use according to any one of claims 1 to 3 wherein the (+)-α-dihydrotetrabenazine or pharmaceutically acceptable salt thereof is administered to the subject once per day.

8. (+)-α-Dihydrotetrabenazine or a pharmaceutically acceptable salt thereof for use according to any one of claims 1 to 3 wherein the (+)-α-dihydrotetrabenazine or pharmaceutically acceptable salt thereof is administered to the subject twice per day.

9. (+)-α-Dihydrotetrabenazine or a pharmaceutically acceptable salt thereof for use according to any one of claims 1 to 3, 7 or 8 wherein the (+)-α-dihydrotetrabenazine or pharmaceutically acceptable salt thereof is administered as a non-extended or delayed release dosage form.

10. (+)-α-Dihydrotetrabenazine or a pharmaceutically acceptable salt thereof for use according to any one of claims 1 to 3 or 7 to 9 wherein the (+)-α-dihydrotetrabenazine or pharmaceutically acceptable salt thereof is not administered in combination with a therapeutically effective amount of amantadine.

11. (+)-α-Dihydrotetrabenazine or a pharmaceutically acceptable salt thereof for use according to any one of claims 1 to 3 or 7 to 10 wherein the (+)-α-dihydrotetrabenazine or pharmaceutically acceptable salt thereof has an isomeric purity of greater than 80%.

## Patentansprüche

1. (+)-α-Dihydrotetrabenazin oder ein pharmazeutisch unbedenkliches Salz davon zur Verwendung beim Behandeln einer hyperkinetischen Bewegungsstörung, wobei die Behandlung Verabreichen einer wirksamen Menge von (+)-α-Dihydrotetrabenazin oder eines pharmazeutischen Salzes davon an ein Subjekt umfasst, wobei die wirksame Menge einer Menge der freien Base von (+)-α-Dihydrotetrabenazin von 0,05 mg/kg bis 0,3 mg/kg pro Tag entspricht.

2. (+)-α-Dihydrotetrabenazin oder ein pharmazeutisch unbedenkliches Salz davon zur Verwendung gemäß Anspruch 1, vorausgesetzt, dass die Gesamtmenge an (+)-α-Dihydrotetrabenazin, die pro Tag verabreicht wird, im Bereich von 0,5 mg bis 20 mg liegt.

3. (+)-α-Dihydrotetrabenazin oder ein pharmazeutisch unbedenkliches Salz davon zur Verwendung gemäß Anspruch 1 oder Anspruch 2, wobei die hyperkinetische Bewegungsstörung ausgewählt ist aus tardiver Dyskinesie, Tourette-Syndrom und Chorea Huntington.

4. (+)-α-Dihydrotetrabenazin oder ein pharmazeutisch unbedenkliches Salz davon zur Verwendung gemäß Anspruch 3, wobei die hyperkinetische Bewegungsstörung Tourette-Syndrom ist.

5. (+)-α-Dihydrotetrabenazin oder ein pharmazeutisch unbedenkliches Salz davon zur Verwendung gemäß Anspruch 3, wobei die hyperkinetische Bewegungsstörung Chorea Huntington ist.

6. (+)-α-Dihydrotetrabenazin oder ein pharmazeutisch unbedenkliches Salz davon zur Verwendung gemäß Anspruch 3, wobei die hyperkinetische Bewegungsstörung tardive Dyskinesie ist.

7. (+)-α-Dihydrotetrabenazin oder ein pharmazeutisch unbedenkliches Salz davon zur Verwendung gemäß einem der Ansprüche 1 bis 3, wobei das (+)-α-Dihydrotetrabenazin oder ein pharmazeutisch unbedenkliches Salz davon dem Subjekt einmal pro Tag verabreicht wird.

8. (+)-α-Dihydrotetrabenazin oder ein pharmazeutisch unbedenkliches Salz davon zur Verwendung gemäß einem der Ansprüche 1 bis 3, wobei das (+)-α-Dihydrotetrabenazin oder das pharmazeutisch unbedenkliche Salz davon dem Subjekt zweimal täglich verabreicht wird.

9. (+)-α-Dihydrotetrabenazin oder ein pharmazeutisch unbedenkliches Salz davon zur Verwendung gemäß einem der Ansprüche 1 bis 3, 7 oder 8, wobei das (+)-α-Dihydrotetrabenazin oder ein pharmazeutisch unbedenkliches Salz davon als eine Dosierungsform mit nicht verlängerter oder verzögerter Freisetzung verabreicht wird.

10. (+)-α-Dihydrotetrabenazin oder ein pharmazeutisch unbedenkliches Salz davon zur Verwendung gemäß einem der Ansprüche 1 bis 3 oder 7 bis 9, wobei das (+)-α-Dihydrotetrabenazin oder ein pharmazeutisch unbedenkliches Salz davon nicht in Kombination mit einer therapeutisch wirksamen Menge Amantadin verabreicht wird.

11. (+)-α-Dihydrotetrabenazin oder ein pharmazeutisch unbedenkliches Salz davon zur Verwendung gemäß einem der Ansprüche 1 bis 3 oder 7 bis 10, wobei das (+)-α-Dihydrotetrabenazin oder das pharmazeutisch unbedenkliche Salz davon eine isomere Reinheit von mehr als 80 % aufweist.

## Revendications

1. (+)-α-dihydrotétrabénazine ou sel acceptable pharmaceutiquement de celle-ci destiné(e) à être utilisé(e) dans le traitement d'un trouble du mouvement hyperkinétique, ledit traitement comprenant l'administration à un sujet d'une quantité efficace de (+)-α-dihydrotétrabénazine ou d'un sel pharmaceutique de celle-ci, ladite quantité efficace correspondant à une quantité de base libre de (+)-α-dihydrotétrabénazine comprise entre 0,05 mg/kg et 0,3 mg/kg par jour.

2. (+)-α-dihydrotétrabénazine ou sel acceptable pharmaceutiquement de celle-ci destiné(e) à être utilisé(e) selon la revendication 1, à condition que la quantité totale de (+)-α-dihydrotétrabénazine administrée par jour soit comprise dans la plage de 0,5 mg à 20 mg.

3. (+)-α-dihydrotétrabénazine ou sel acceptable pharmaceutiquement de celle-ci destiné(e) à être utilisé(e) selon la revendication 1 ou la revendication 2, ledit trouble du mouvement hyperkinétique étant choisi parmi la dyskinésie tardive, le syndrome de la Tourette et la maladie de Huntington.

4. (+)-α-dihydrotétrabénazine ou sel acceptable pharmaceutiquement de celle-ci destiné(e) à être utilisé(e) selon la revendication 3, ledit trouble du mouvement hyperkinétique étant le syndrome de la Tourette.

5. (+)-α-dihydrotétrabénazine ou sel acceptable pharmaceutiquement de celle-ci destiné(e) à être utilisé(e) selon la revendication 3, ledit trouble du mouvement hyperkinétique étant la maladie de Huntington.

6. (+)-α-dihydrotétrabénazine ou sel acceptable pharmaceutiquement de celle-ci destiné(e) à être utilisé(e) selon la revendication 3, ledit trouble du mouvement hyperkinétique étant la dyskinésie tardive.

7. (+)-α-dihydrotétrabénazine ou sel acceptable pharmaceutiquement de celle-ci destiné(e) à être utilisé(e) selon l'une quelconque des revendications 1 à 3, ladite (+)-α-dihydrotétrabénazine ou ledit sel acceptable pharmaceutiquement de celle-ci étant administré(e) au sujet une fois par jour.

8. (+)-α-dihydrotétrabénazine ou sel acceptable pharmaceutiquement de celle-ci destiné(e) à être utilisé(e) selon l'une quelconque des revendications 1 à 3, ladite (+)-α-dihydrotétrabénazine ou ledit sel acceptable pharmaceutiquement de celle-ci étant administré(e) au sujet deux fois par jour.

9. (+)-α-dihydrotétrabénazine ou sel acceptable pharmaceutiquement de celle-ci destiné(e) à être utilisé(e) selon l'une quelconque des revendications 1 à 3, 7 ou 8, ladite (+)-α-dihydrotétrabénazine ou ledit sel acceptable pharmaceutiquement de celle-ci étant administré(e) sous une forme posologique à libération non prolongée ou retardée.

10. (+)-α-dihydrotétrabénazine ou sel acceptable pharmaceutiquement de celle-ci destiné(e) à être utilisé(e) selon l'une quelconque des revendications 1 à 3 ou 7 à 9, ladite (+)-α-dihydrotétrabénazine ou ledit sel acceptable pharmaceutiquement de celle-ci n'étant pas administré en combinaison avec une quantité thérapeutiquement efficace d'amantadine.

11. (+)-α-dihydrotétrabénazine ou sel acceptable pharmaceutiquement de celle-ci destiné(e) à être utilisé(e) selon l'une quelconque des revendications 1 à 3 ou 7 à 10, ladite (+)-α-dihydrotétrabénazine ou ledit sel acceptable pharmaceutiquement de celle-ci présentant une pureté isomérique supérieure à 80 %.
